# EUROPEAN PATENT APPLICATION

(11) **EP 4 455 303 A1**
(43) Date of publication of application: **30.10.2024**
(21) Application number: 23315135.6
(22) Date of filing: 28.04.2023
(51) Int. Cl.: C12Q 1/6848, G16B 25/10, G16B 25/20

(54) **NUCLEIC ACID AMPLIFICATION PROCESS CONTROLS**

(71) Applicant: Mobidiag Oy, 02150 Espoo (FI)
(72) Inventor: Hennetin, Jérôme, 75011 PARIS (FR); Bondet, Jean-Sebastien, 75011 PARIS (FR)
(74) Representative: Regimbeau

(57) **Abstract**

Disclosed are methods, systems, reaction mixtures, kits, apparatuses, and systems for monitoring nucleic acid amplification reaction mixtures for abnormalities. Exemplary methods comprise determining fluorescence-based values from fluorophore-containing nucleic acid amplification reaction mixtures and determining whether those values satisfy predetermined values or indicate abnormalities.

## Description

### FIELD

This disclosure relates to the field of nucleic acid amplification. More particularly, the disclosure concerns methods, materials, apparatuses, and systems for monitoring nucleic acid amplification reaction mixtures for abnormalities during an amplification process.

### INTRODUCTION

Nucleic acid amplification reactions are widely used in research and clinical laboratories for detecting genetic disorders and infectious diseases. Nucleic acid amplification reactions utilize enzymes such as polymerases and ligases, separately or in combination, to generate multiple copies of a target nucleic acid sequence in primer extension reactions which incorporate nucleotides or by ligation of adjacent probes that are complementary to a target nucleic acid sequence. In such reactions, each template generates more copies, and the copies may themselves become templates. (The nucleic acid copies are referred to as "amplicon.") In a nucleic acid amplification reaction, a reaction mixture may be subjected to a number of thermal cycles ("PCR cycles"), each of which includes a denaturation step, a primer annealing step, and a primer extension step. In the denaturation step, a double-stranded DNA template molecule is made single-stranded; in the primer annealing step, primers bind to complementary sequences in the single-stranded DNA template; and in the primer extension step, new DNA strands are formed from the primers. By selecting an appropriate temperature for each step of a PCR cycle, the PCR cycle can be repeated until there is a sufficient amount of amplicon in a reaction mixture to render the target nucleic acid sequence detectable or quantifiable. During the early stages of PCR, the amplification is exponential.

Nucleic acid amplification reactions rely on the accuracy of the temperatures and buffer compositions of the reaction mixtures. Amplification of the target nucleic acids is mainly performed by an enzymatic method using DNA polymerase (for DNA target amplification) or DNA polymerase and reverse transcriptase (for RNA target amplification). These enzymes are sensitive to changes in temperature and reaction conditions, such as pH. To maintain enzyme stability, nucleic acid amplification reactions may include Tris and sulphate compounds (such as MgSO₄, NH₄SO₄).

Temperatures and buffer compositions that deviate from what is normal or expected can arise during reaction mixture preparation, conditions and/or may be caused by errors in the functionality of nucleic acid amplification systems. Such deviations can lead to unreliable or inconsistent results and even complete failure of a nucleic acid amplification reaction. Typically, whether a nucleic amplification reaction is a success or failure cannot be determined until after a reaction is completed or should have been completed, if at all, and a control reaction is generally necessary to reliably detect problems with buffer and/or cycling conditions. The absence of amplification (of either a target nucleic acid, if present in a reaction mixture, or a control) is a clear indication of failure but generally does not indicate the cause of the failure. Disclosed herein are materials, methods, and systems for detecting abnormalities that relate to such errors in process. The methods disclosed herein can rely on the responsiveness of oligonucleotide primers, fluorophores, and quenchers to changes in the temperature of reaction mixtures.

### SUMMARY

The following embodiments are among those provided by the disclosure.

Embodiment 1 is a method of monitoring a nucleic acid amplification reaction mixture for an abnormality, the method comprising:
a) providing a fluorophore-containing nucleic acid amplification reaction mixture in a nucleic acid amplification system;
b) adjusting a heating element of the nucleic acid amplification system to a first temperature for a first time period, thereby heating the nucleic acid amplification reaction mixture to a first incubation temperature during the first period;
c) after step b), measuring fluorescence from the fluorophore at a plurality of times during the first time period, wherein the nucleic acid amplification reaction mixture is not exposed to nucleic acid amplification conditions prior to or during the first time period;
d) determining:
   i) a first value representative of a change in fluorescence signal during the first time period; and/or
   ii) a second value representative of the rate of change in fluorescence during the first time period; and
e) comparing the first value to a first predetermined threshold or range, and/or comparing the second value to a second predetermined threshold or range, wherein an abnormality is detected if
   i) the first value indicates a change in fluorescence that does not satisfy the first predetermined threshold or that is outside the first predetermined range;
   ii) the second value indicates a rate of temperature change that does not satisfy the second predetermined threshold or that is outside the second predetermined range;
   iii) either the first value indicates a change in fluorescence that does not satisfy the first predetermined threshold or that is outside the first predetermined range, or the second value indicates a rate of temperature change that does not satisfy the second predetermined threshold or that is outside the second predetermined range; or
   iv) the first value indicates a change in fluorescence that does not satisfy the first predetermined threshold or that is outside the first predetermined range, and the second value indicates a rate of temperature change that does not satisfy the second predetermined threshold or that is outside the second predetermined range.

Embodiment 2 is a nucleic acid amplification system comprising: a docking station configured to receive a reaction vessel for containing at least one nucleic acid amplification mixture; a heating element disposed in proximity to a position to be occupied by a nucleic acid amplification mixture when the reaction vessel is present in the docking station; a fluorescence detector configured to measure fluorescence of a fluorophore in the nucleic acid mixture; and a processor operably linked to the heating element and a memory;
the memory comprising instructions that when executed by the processor cause the nucleic acid amplification system to perform a method of monitoring a nucleic acid amplification reaction mixture comprising a fluorophore for an abnormality, the method comprising:
a) adjusting the heating element to a first temperature for a first time period;
b) after step a), measuring fluorescence with the fluorescence detector from a fluorophore contained in the nucleic acid amplification mixture at a plurality of times during the first time period, wherein the first time period occurs before a scheduled nucleic acid amplification;
c) determining
   i) a first value representative of a change in fluorescence signal during the first time period; and/or
   ii) a second value representative of the rate of change in fluorescence during the first time period; and
d) comparing the first value to a first predetermined threshold or range, and/or comparing the second value to a second predetermined threshold or range, wherein an abnormality is detected if
   i) the first value indicates a change in fluorescence that does not satisfy the first predetermined threshold or that is outside the first predetermined range;
   ii) the second value indicates a rate of temperature change that does not satisfy the second predetermined threshold or that is outside the second predetermined range;
   iii) either the first value indicates a change in fluorescence that does not satisfy the first predetermined threshold or that is outside the first predetermined range, or the second value indicates a rate of temperature change that does not satisfy the second predetermined threshold or that is outside the second predetermined range; or
   iv) the first value indicates a change in fluorescence that does not satisfy the first predetermined threshold or that is outside the first predetermined range and the second value indicates a rate of temperature change that does not satisfy the second predetermined threshold or that is outside the second predetermined range.

Embodiment 3 is a computer-readable medium comprising: instructions that when executed by a processor of a nucleic acid amplification system cause the nucleic acid amplification system to perform a method of monitoring a nucleic acid amplification reaction mixture comprising a fluorophore for an abnormality, the method comprising:
a) adjusting a heating element of the nucleic acid amplification system to a first temperature for a first time period;
b) after step a), measuring fluorescence with a fluorescence detector of the nucleic acid amplification system from a fluorophore contained in a nucleic acid amplification mixture at a plurality of times during thé first time period, wherein the first time period occurs before a scheduled nucleic acid amplification;
c) determining
   i) a first value representative of a change in fluorescence signal during the first time period; and/or
   ii) a second value representative of the rate of change in fluorescence during the first time period; and
d) comparing the first value to a first predetermined threshold or range, and/or comparing the second value to a second predetermined threshold or range, wherein an abnormality is detected if
   i) the first value indicates a change in fluorescence that does not satisfy the first predetermined threshold or that is outside the first predetermined range;
   ii) the second value indicates a rate of temperature change that does not satisfy the second predetermined threshold or that is outside the second predetermined range;
   iii) either the first value indicates a change in fluorescence that does not satisfy the first predetermined threshold or that is outside the first predetermined range, or the second value indicates a rate of temperature change that does not satisfy the second predetermined threshold or that is outside the second predetermined range; or
   iv) the first value indicates a change in fluorescence that does not satisfy the first predetermined threshold or that is outside the first predetermined range and the second value indicates a rate of temperature change that does not satisfy the second predetermined threshold or that is outside the second predetermined range.

Embodiment 4 is the method, system, or computer-readable medium of any one of the preceding embodiments, wherein the first value is determined by subtracting an earlier measurement of fluorescence from the fluorophore from a later measurement of fluorescence from the fluorophore.

Embodiment 5 is the method, system, or computer-readable medium of the immediately preceding embodiment, wherein the earlier measurement of fluorescence from the fluorophore is obtained before or during the first half of the first time period, within one minute of the beginning of the first time period, or at about the beginning of the first time period.

Embodiment 6 is the method, system, or computer-readable medium of any one of embodiments 4-5, wherein the earlier measurement of fluorescence from the fluorophore is the lowest measurement of fluorescence from the fluorophore.

Embodiment 7 is the method, system, or computer-readable medium of any one of embodiments 4-6, wherein the later measurement of fluorescence from the fluorophore is obtained during the second half of the first time period, within one minute of the end of the first time period, or at about the end of the first time period.

Embodiment 8 is the method, system, or computer-readable medium of any one of embodiments 4-7, wherein the later measurement of fluorescence from the fluorophore is the highest measurement of fluorescence from the fluorophore.

Embodiment 9 is the method, system, or computer-readable medium of any one of the preceding embodiments, wherein
a) the second value is determined by dividing a first subvalue by a second subvalue;
b) the first subvalue, represents a lower area of a quadrilateral region of a plot of fluorescence from the fluorophore versus time during the first time period; and
c) the second subvalue represents an upper area of the quadrilateral region, the quadrilateral region being bounded by
   i) first and last measurements of fluorescence from the fluorophore during the first time period and the times of the first and last measurements of fluorescence from the fluorophore during the first time period;
   ii) minimum and maximum measurements of fluorescence from the fluorophore during the first time period and the times of the minimum and maximum measurements of fluorescence from the fluorophore during the first time period;
   iii) minimum and maximum measurements of fluorescence from the fluorophore during the first time period and the times of first and last measurements of fluorescence from the fluorophore during the first time period; or
   iv) first and last measurements of fluorescence from the fluorophore during the first time period and the times of minimum and maximum measurements of fluorescence from the fluorophore during the first time period;
   wherein the lower area equals the area of the quadrilateral region below the measurements of fluorescence from the fluorophore and the upper area equals the area of the quadrilateral region above the measurements of fluorescence from the fluorophore.

Embodiment 10 is the method, system, or computer-readable medium of any one of the preceding embodiments, wherein the first value represents Y-axis change in fluorescence signal.

Embodiment 11 is the method, system, or computer-readable medium of any one of the preceding embodiments, wherein the fluorescence changes over time during the first time period, and the rate of fluorescence change during the first time period correlates with a rate of temperature change in the nucleic acid amplification reaction mixture.

Embodiment 12 is the method, system, or computer-readable medium of any one of the preceding embodiments, wherein the abnormality is defective thermal contact and/or defective liquid handling.

Embodiment 13 is the method, system, or computer-readable medium of any one of the preceding embodiments, wherein nucleic acid in the fluorophore-containing nucleic acid amplification reaction mixture is denatured during the first time period.

Embodiment 14 is the method, system, or computer-readable medium of any one of the preceding embodiments, wherein the first time period is in the range of about 2-10 minutes, 3-9 minutes, 3-7 minutes, or 4-6 minutes, or is about 5 minutes.

Embodiment 15 is the method, system, or computer-readable medium of any one of the preceding embodiments, wherein the first temperature is in the range of 90°C-120°C or 95°C-115°C, or is about 110°C.

Embodiment 16 is the method, system, or computer-readable medium of any one of the preceding embodiments, wherein the method further comprises adjusting the heating element to a preliminary temperature for a preliminary time period before step a).

Embodiment 17 is the method, system, or computer-readable medium of the immediately preceding embodiment, wherein the preliminary temperature is suitable for reverse transcription.

Embodiment 18 is the method, system, or computer-readable medium of the immediately preceding embodiment, wherein reverse transcription occurs in the fluorophore-containing nucleic acid amplification reaction mixture during the preliminary time period.

Embodiment 19 is the method, system, or computer-readable medium of any one of embodiments 16-18, wherein the preliminary temperature is in the range of 25°C to 50°C, 30°C to 50°C, 35°C to 50°C, 40°C to 50°C, or 42°C to 49°C, or is about 48°C.

Embodiment 20 is the method, system, or computer-readable medium of any one of embodiments 16-18, wherein the preliminary time period is in the range of 15-60 minutes, 20-50 minutes, or 25-40 minutes, or is about 30 minutes.

Embodiment 21 is the method, system, or computer-readable medium of any one of the preceding embodiments, wherein the fluorophore-containing nucleic acid amplification reaction mixture is a thermocycled reaction mixture.

Embodiment 22 is the method, system, or computer-readable medium of any one of the preceding embodiments, wherein the fluorophore-containing nucleic acid amplification reaction mixture is a PCR or RT-PCR reaction mixture.

Embodiment 23 is the method, system, or computer-readable medium of any one of the preceding embodiments, wherein the fluorophore-containing nucleic acid amplification reaction mixture is contained in a microfluidic cartridge.

Embodiment 24 is the method, system, or computer-readable medium of the immediately preceding embodiment, wherein the microfluidic cartridge comprises:
a) a plurality of functional areas comprising a sample preparation area, a nucleic acid amplification area, and a waste area;
b) a central distribution hub; and
c) a pump, a plurality of valves, and a fluidic network of microchannels connecting the functional areas to the hub;
wherein the pump, plurality of valves, and fluidic network of microchannels can drive movement of a fluid from a first functional area through the central distribution hub to a second functional area of the plurality of functional areas.

Embodiment 25 is the method, system, or computer-readable medium of the immediately preceding embodiment, wherein the fluorophore-containing nucleic acid amplification reaction mixture is located in a nucleic acid amplification area during the first time period.

Embodiment 26 is the method, system, or computer-readable medium of embodiment 25, wherein the method further comprises adjusting the heating element to a preliminary temperature for a preliminary time period before step a) and the fluorophore-containing nucleic acid amplification reaction mixture is located in the nucleic acid amplification area during the preliminary time period.

Embodiment 27 is the method, system, or computer-readable medium of any one of the preceding embodiments, wherein a plurality of fluorophore-containing nucleic acid amplification reaction mixtures is provided in the nucleic acid amplification system in step a).

Embodiment 28 is the method, system, or computer-readable medium of the immediately preceding embodiment, wherein fluorescence is measured from the fluorophores of each of the plurality of fluorophore-containing nucleic acid amplification reaction mixtures in step c).

Embodiment 29 is the method, system, or computer-readable medium of the immediately preceding embodiment, wherein
a) the first value representative of a change in fluorescence signal during the first time period, and/or
b) the second value representative of the rate of change in fluorescence during the first time period,
is determined for each of the plurality of fluorophore-containing nucleic acid amplification reaction mixtures in step d).

Embodiment 30 is the method, system, or computer-readable medium of the immediately preceding embodiment, wherein for each of the plurality of fluorophore-containing nucleic acid amplification reaction mixtures, the first value is compared to a first predetermined threshold or range and/or the second value is compared to a second predetermined threshold or range in step e).

Embodiment 31 is the method, system, or computer-readable medium of any one of embodiments 27-30, wherein the plurality of fluorophore-containing nucleic acid amplification reaction mixtures is contained in a multi-well plate or in a plurality of tubes.

Embodiment 32 is the method, system, or computer-readable medium of any one of the preceding embodiments, wherein an abnormality is detected if the first value indicates a change in fluorescence that does not satisfy the first predetermined threshold or that is outside the first predetermined range.

Embodiment 33 is the method, system, or computer-readable medium of any one of embodiments 1-28, wherein an abnormality is detected if the second value indicates a rate of temperature change that does not satisfy the second predetermined threshold or that is outside the second predetermined range.

Embodiment 34 is the method, system, or computer-readable medium of any one of embodiments 1-28, wherein an abnormality is detected if either the first value indicates a change in fluorescence that does not satisfy the first predetermined threshold or that is outside the first predetermined range or the second value indicates a rate of temperature change that does not satisfy the second predetermined threshold or that is outside the second predetermined range.

Embodiment 35 is the method, system, or computer-readable medium of any one of embodiments 1-28, wherein an abnormality is detected if the first value indicates a change in fluorescence that does not satisfy the first predetermined threshold or that is outside the first predetermined range and the second value indicates a rate of temperature change that does not satisfy the second predetermined threshold or that is outside the second predetermined range.

Embodiment 36 is the method, system, or computer-readable medium of any one of the preceding embodiments, wherein the fluorophore is associated with an oligonucleotide probe, optionally wherein the oligonucleotide probe further comprises a quencher.

Embodiment 37 is the method, system, or computer-readable medium of any one of the preceding embodiments, wherein the adjusting the heating element to the first temperature for the first time period is a reverse transcriptase inactivation step.

Embodiment 38 is the method, system, or computer-readable medium of any one of the preceding embodiments, wherein the fluorophore is temperature-sensitive.

Embodiment 39 is the method, system, or computer-readable medium of any one of the preceding embodiments, wherein the fluorophore is sulforhodamine.

Embodiment 40 is the method of any one of embodiments 1 or 4-39, wherein an abnormality is detected and the nucleic acid amplification reaction mixture is not subjected to nucleic acid amplification conditions.

Embodiment 41 is the method of any one of embodiments 1 or 4-39, wherein no abnormality is detected and the nucleic acid amplification reaction mixture is subjected to nucleic acid amplification conditions.

Embodiment 42 is the system of any one of embodiments 2 or 4-39, wherein the system is configured not to subject the nucleic acid amplification reaction mixture to nucleic acid amplification conditions if an abnormality is detected and/or is configured to subject the nucleic acid amplification reaction mixture to nucleic acid amplification conditions only if an abnormality is not detected.

Embodiment 43 is the system or computer-readable medium of any one of embodiments 2, 3, 4-39, or 42, wherein the method further comprises subjecting the nucleic acid amplification reaction mixture to nucleic acid amplification conditions only if an abnormality is not detected.

Embodiment 44 is the system or computer-readable medium of any one of embodiments 2, 3, 4-39, or 42-43, wherein the method further comprises reducing the temperature of the heating element without thermocycling if an abnormality is detected.

Additional objects and advantages will be set forth in part in the description which follows, and in part will be understood from the description, or may be learned by practice. The objects and advantages will be realized and attained by means of the elements and combinations particularly pointed out in the appended claims.

It is to be understood that both the foregoing general description and the following detailed description are exemplary and explanatory only and are not restrictive of the claims.

### BRIEF DESCRIPTION OF THE DRAWINGS

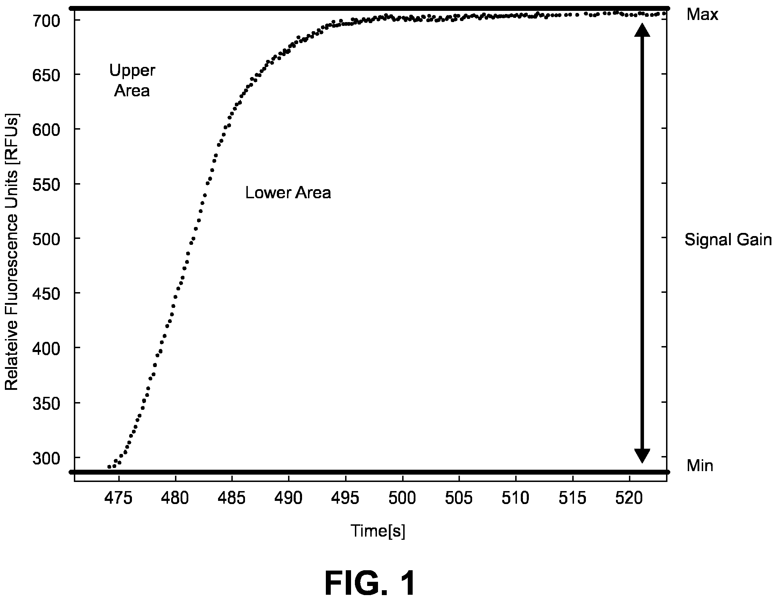

Figure 1 is an exemplary curve for a single nucleic acid amplification reaction for determining signal gain and thermal ratio from a first time period. Relative fluorescence units (RFUs; y-axis) are plotted over time (milliseconds; x-axis). Signal gain is the difference between RFU Max and RFU Min for the time period shown. Thermal ratio is the ratio of the lower area of the quadrilateral region to the upper area of the quadrilateral region.

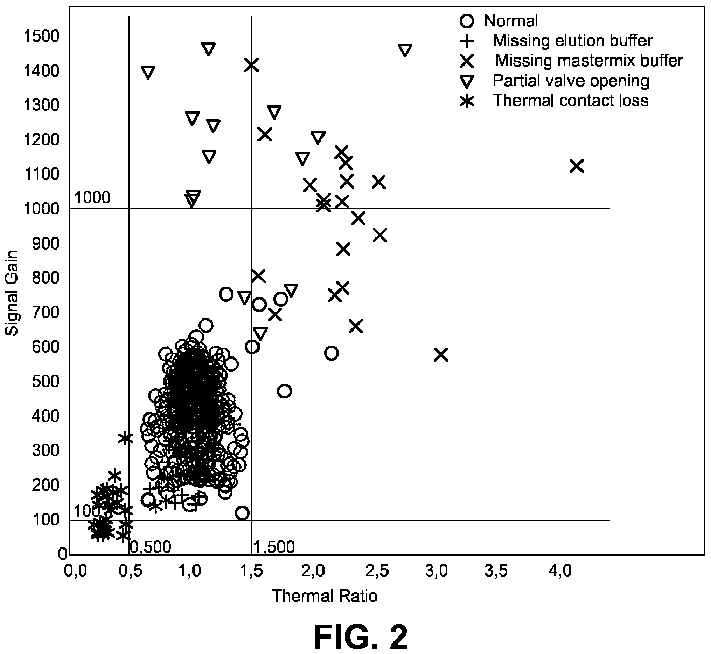

Figure 2 is an exemplary plot of signal gain (y-axis) versus thermal ratio (x-axis) for a plurality of nucleic acid amplification reactions performed under different conditions, including normal conditions, thermal contact loss, and fluidic errors, such as missing elution buffer, missing master mix buffer, and partial valve opening.

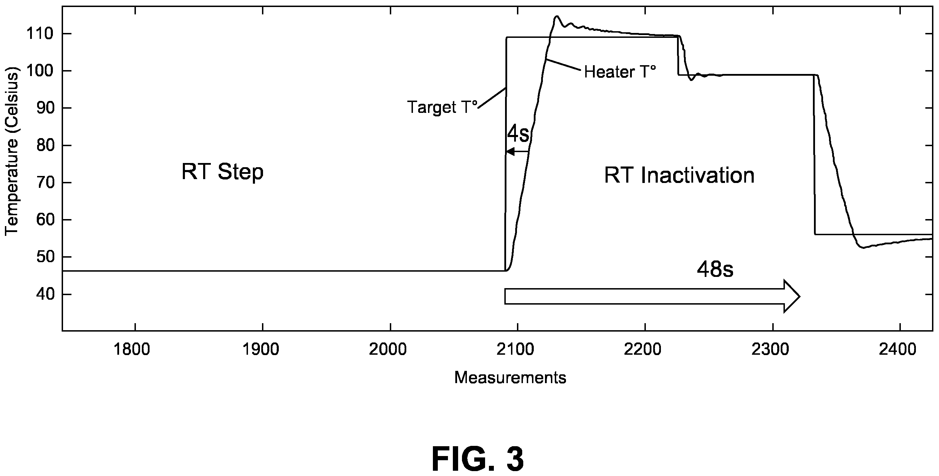

Figure 3 is an exemplary plot of PCR module target and heater temperature curves logged during a nucleic acid amplification reaction. The curve is partitioned to identify a reverse transcription (RT) step and an RT inactivation phase. The limit of the RT inactivation phase can be determined by the target temperature or by the heater temperature. The time period includes a delay of 4 seconds to allow the temperature' to cross the target temperature of 80°C.

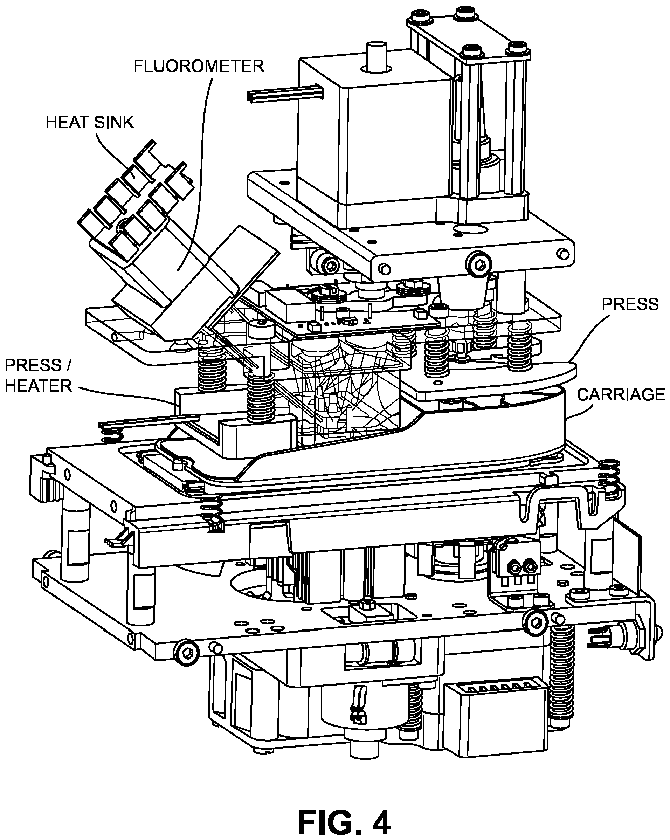

Figure 4 is an exemplary nucleic acid amplification system docking station loaded with a microfluidic cartridge. The system includes a heat sink, a fluorometer, and a press/ heater used for interacting and measuring a sample loaded in the cartridge.

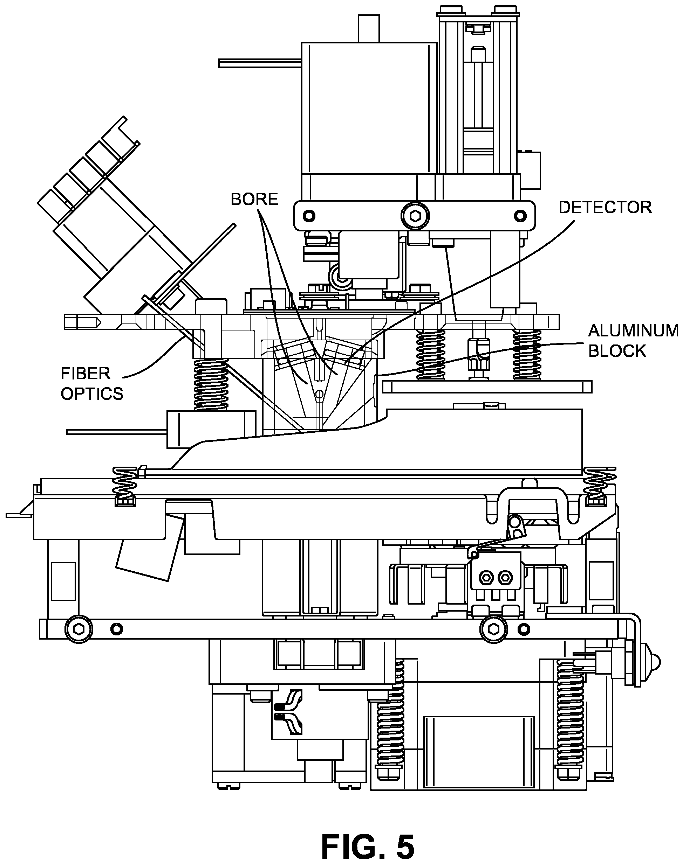

Figure 5 is a side view of the docking station of Figure 4. The side view shows the fiber optics that run from the cartridge to the fluorometer as well as the detector, bore and aluminum block used for taking fluorescence measurements.

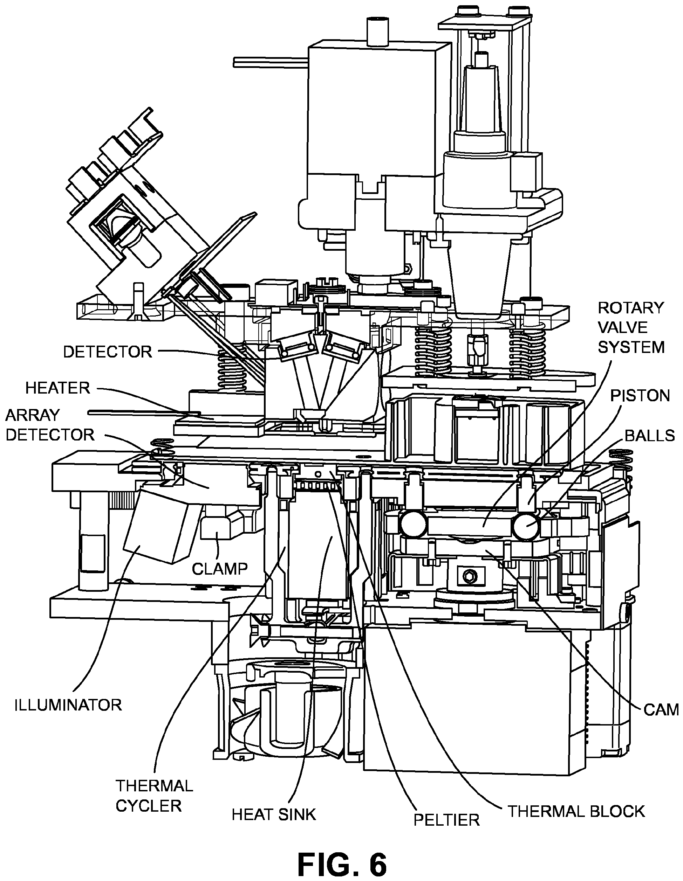

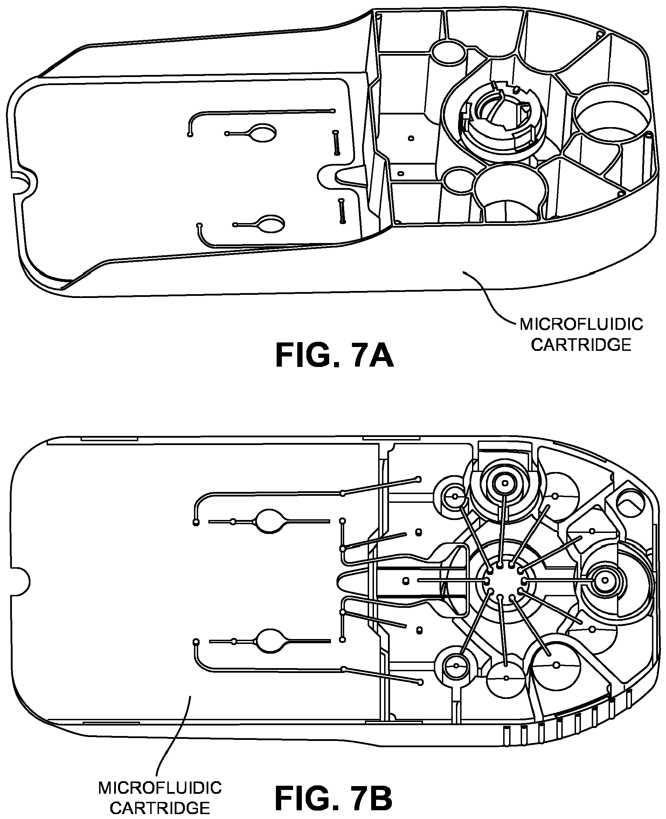

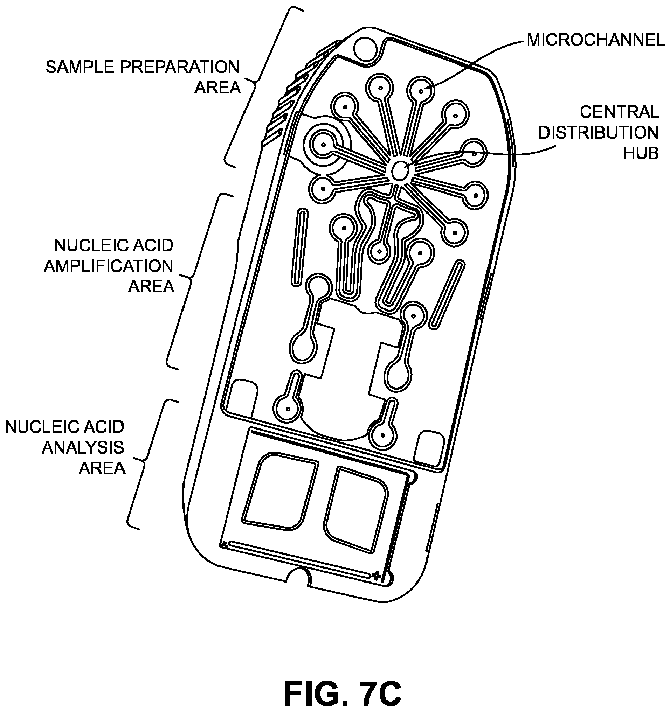

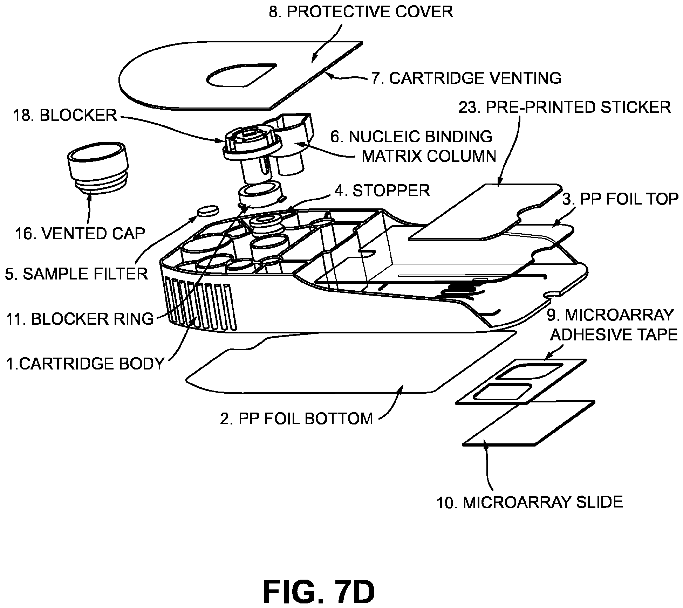

Figure 6 shows a cutaway view of the nucleic acid amplification system docking station and a microfluidic cartridge of Figures 7 and 8. The cutaway view shows internal components such as the thermal cycler, thermal block, array detector, rotary valve system and pistons useful for moving and analyzing liquids in different functional areas of the cartridge.
Figures 7A-7D show various views of an exemplary microfluidic cartridge. Figure 7A shows a top-down view of a microfluidic cartridge which contains multiple chambers for housing samples, reagents, or other liquids. Figure 7B shows the bottom view of the exemplary microfluidic cartridge which shows a fluidic network of microchannels connecting various chambers to other areas within the cartridge. Figure 7C shows a bottom view of a fully constructed microfluidic cartridge which includes a sample preparation area, a nucleic acid amplification area, and a nucleic acid analysis area. These functional areas are connected by the series of microchannels shown in detail in Figure 7B. The sample preparation area includes liquids in adjacent chambers. The system can be programmed to combine liquids contained in these chambers in discrete volumes in a particular order. When docked in the system, the nucleic acid amplification area is in close proximity to the thermal cycler in Figure 5. Similarly, when docked in the system, the nucleic acid analysis area is in close proximity to the array detector as shown in Figure 6. Figure 7D shows an exploded view of the fully constructed exemplary microfluidic cartridge which includes a cartridge body (1) a vented cap for covering the sample after input (16), a sample filter (5) and the components for the cartridge to interact with the mechanical components of the system to move and combine liquids in the sample preparations area (4, 6-8, 11). The exploded view also shows how the microarray slide (10) is attached to the nucleic acid amplification area via an adhesive tape (9) in the nucleic amplification analysis area. The cartridge also includes polypropylene (PP) foil coverings (2, 3).

### DEFINITIONS

Unless defined otherwise, all technical and scientific terms used herein have the same meaning as commonly understood by one of ordinary skill in the art pertinent to the methods and compositions described. All patents, applications, published applications and other publications referred to herein are incorporated by reference in their entirety. If a definition set forth in this section is contrary to or otherwise inconsistent with a definition set forth in the patents, applications, published applications, and other publications that are herein incorporated by reference, the definition set forth in this section prevails over the definition that is incorporated herein by reference.

"Nucleic acid" and "polynucleotide" refer to a multimeric compound including two or more covalently bonded nucleosides or nucleoside analogs having nitrogenous heterocyclic bases, or base analogs, where the nucleosides are linked together by phosphodiester bonds or other linkages to form a polynucleotide. Nucleic acids include RNA, DNA, and combinations and analogs thereof such as "peptide nucleic acids" or PNAs (see, e.g., International Pub. No. WO 95/32305) and "locked nucleic acids" (LNA), in which one or more nucleotide monomers have a bicyclic furanose unit locked in an RNA mimicking sugar conformation (see, e.g., Vester et al., Biochemistry 43:13233-41, 2004). Nitrogenous bases may be conventional bases (A, G, C, T, U), analogs thereof (e.g., inosine, 5-methylisocytosine, isoguanine; see, e.g., The Biochemistry of the Nucleic Acids 5-36, Adams et al., ed., 11th ed., 1992; Abraham et al., 2007, BioTechniques 43: 617-24), which include derivatives of purine or pyrimidine bases (e.g., N4-methyl deoxyguanosine, deaza- or aza-purines, deaza- or azapyrimidines, etc.; U.S. Pat. Nos. 5,378,825 and 6,949,367 and International Pub. No. WO 93/13121), and/or "abasic" residues (see. e.g., U.S. Pat. No. 5,585,481).

A "target" material as used herein, is a material to be detected or quantified. A target material may be a nucleic acid; other embodiments of target materials include cells, viruses, and other biomolecules. A "target nucleic acid" as used herein is a nucleic acid including a target sequence to be detected or quantified, e.g., via amplification. Target nucleic acids or target nucleic acids of interest may be DNA or RNA or combinations or analogs thereof as described herein and may be either single-stranded or double-stranded. The target nucleic acid may include other sequences besides the target sequence, which may not be detected or quantified.

The term "region," as used herein, refers to a portion of a nucleic acid, where the portion is less than the entire nucleic acid. For example, the term "region" may be used to refer to a smaller target-hybridizing portion of the entire oligonucleotide. Certain oligonucleotides, such as primers, may consist entirely of one region (e.g., target-hybridizing region) or may contain a plurality of regions (e.g., a promoter sequence region and a target-hybridizing region).

"Primer," "amplification oligonucleotide" or "oligonucleotide primer" refers to a polynucleotide, generally an oligonucleotide including a "target" binding portion, that is designed to selectively hybridize with a target nucleic acid flanking sequence or to a corresponding primer-binding site of an amplification product under appropriate stringency conditions and serve as the initiation point for the synthesis of a nucleotide sequence that is complementary to the corresponding polynucleotide template, e.g., from its 3' end. The 5' region of the primer may be non-complementary to the target nucleic acid. If the 5' non-complementary region includes a promoter sequence, it is referred to as a "promoter-primer." Promoter-primers may serve as a primer for a DNA polymerase, which extends the promoter-primer from its 3' end, or as a promoter for an RNA polymerase, which initiates de novo RNA synthesis at or near the promoter sequence contained in the promoter-primer.

"Sample" refers to any material that may contain or is suspected of containing target nucleic acids. A sample may be a complex mixture of components. Samples include "biological samples" which include any tissue or material derived from a living or dead mammal or organism, including, for example, stool, blood, plasma, serum, blood cells, saliva, mucous and cerebrospinal fluid. Samples may also include samples of *in vitro* cell culture constituents including, for example, conditioned media resulting from the growth of cells and tissues in culture medium. Samples also include food, which includes any material intended or suitable for consumption, including solids, suspensions, emulsions, gels, and liquids (thus including gelatin, milk, soups, beverages, ice-cream, fruit smoothies, emulsified cheese dips, fruit puree, nut butter, processed and/or textured protein, and the like, as well as bread, fruit, vegetables, and meat). Samples also include water and aqueous solutions. A sample may be treated chemically, physically, or mechanically to disrupt tissue or cell structure to release intracellular nucleic acids into a solution. Samples may be treated to release nucleic acids into a solution containing enzymes, buffers, salts, detergents and the like.

The interchangeable terms "oligomer," "oligo," and "oligonucleotide" refer to a nucleic acid having generally less than 1,000 nucleotide (nt) residues, including polymers in a range having a lower limit of about 5 nt residues and an upper limit of about 500 to about 900 nt residues. In some embodiments, oligonucleotides are in a size range having a lower limit of about 12 to about 15 nt and an upper limit of about 50 to about 600 nt, and other embodiments are in a range having a lower limit of about 15 to 20 nt and an upper limit of about 22 to about 100 nt. An oligonucleotide may serve one or more of various different functions, e.g., as a primer and/or promoter, detection probe, capture oligomer, etc.

"Amplifying" or "amplification" refers to any known procedure for obtaining multiple copies of a target nucleic acid sequence or its complement or fragments thereof. The multiple copies may be referred to as amplicons or amplification products. As used herein, the term "nucleic acid amplification conditions" refers to both temperature and chemical conditions that enable nucleic acid amplification. Methods of forming an reaction mixture, and subjecting the reaction mixture to conditions suitable for nucleic acid amplification are well established. Known amplification methods include both thermal cycling and isothermal amplification methods. Polymerase chain reaction (PCR), replicase-mediated amplification, ligase chain reaction (LCR), strand-displacement amplification (SDA), and transcription-associated amplification (e.g., transcription-mediated amplification (TMA) or NASBA) are non-limiting examples of nucleic acid amplification methods. See, e.g., U.S Pat. Nos. 4,868,105, 5,124,246, 5,130,238, 5,399,491, 5,437,990, 5,554,516 and 7,374,885, and International Pub. Nos. WO 88/01302, WO 88/10315 and WO 95/03430 (TMA); U.S. Pat. No. 4,786,600 (RCA); U.S. Pat. Nos. 5,427,930 and 5,516,663 (LCR); and U.S. Pat. No. 5,422,252, 5,547,861 and5,648,211 (SDA). See also, e.g., Compton, Nature 350:91-92, 1991; Malek et al., Methods Mol. Biol. 28:253-260, 1994 (NASBA). PCR is the preferred amplification method, and is well-known in the art. Briefly, PCR amplification uses a DNA polymerase, pairs of primers, and thermal cycling to synthesize multiple copies of two complementary strands from dsDNA or from a cDNA (see, e.g., US Pat. Nos. 4,683,195, 4,683,202, and 4,800,159).

As used herein, the term "real-time amplification" refers to amplification of target nucleic acid that is monitored by real-time detection means. Real-time PCR amplification includes, e.g., what is commonly referred to as TaqMan^{®} PCR (see, e.g., Holland et al., Proc. Natl. Acad. Sci. USA 88:7276-7280, 1991; and Livak et al., U.S. Pat. No. 6,030,787). TaqMan PCR is a type of real-time PCR that uses a nucleic acid probe complementary to an internal segment of the target DNA. The probe may be labeled with two fluorescent moieties. The emission spectrum of one overlaps the excitation spectrum of the other, resulting in "quenching" of the first fluorophore by the second. In some applications, a fluorescent moiety may be used in combination with a non-fluorescent quencher moiety.

As used herein, "thermocyling" is a process of cyclically heating and cooling of a nucleic acid amplification mixture that facilitates amplifying nucleic acids through successive rounds of denaturation, primer annealing, and primer extension, e.g., by a thermostable polymerase. In many examples, thermocycling includes holding a reaction mixture at two or more different temperatures, each for a predetermined length of time, and performing several cycles of those two or more temperatures to cause nucleic acid amplification. A nucleic acid amplification reaction mixture that has been subjected to thermocycling is termed a "thermocycled reaction mixture."

As used herein, a nucleic acid amplification system refers to a device or apparatus that may be used to perform nucleic acid amplification. In many examples, the nucleic acid amplification system includes a temperature controller and is capable of providing or transferring heat to nucleic acid amplification reaction mixtures via one or more heating elements, which may include, e.g., a heat block. The nucleic acid amplification system is typically programmable and can hold temperatures for different lengths of time. The nucleic acid amplification system may be configured to accommodate any one or more of a variety of reaction vessels, such as tubes, multi-well strips, multi-well plates, microfluidic chips, and microfluidic cartridges, which may contain one or more nucleic acid amplification mixtures.

The term "amplicon" or the term "amplification product" as used herein refers to the nucleic acid molecule generated during an amplification procedure that is complementary or homologous to a sequence contained within the target sequence. These terms can be used to refer to a single-stranded amplification product, a double-stranded amplification product, or one of the strands of a double-stranded amplification product.

By "complementary" is meant that the nucleotide sequences of similar regions of two single-stranded nucleic acids, or two different regions of the same single-stranded nucleic acid, have nucleotide base compositions that allow the single-stranded regions to hybridize together in a stable double-stranded hydrogen-bonded region under stringent hybridization or amplification conditions. "Stringent" hybridization or amplification conditions selectively enable the hybridization of highly homologous nucleic acid sequences. Such conditions may include high hybridization temperatures and a low concentration of salt in the buffers. Sequences that hybridize to each other may be completely complementary or partially complementary by standard nucleic acid base pairing (e.g., G:C, A:T, or A:U pairing). By "sufficiently complementary" is meant a contiguous sequence that is capable of hybridizing to another sequence by hydrogen bonding between a series of complementary bases, which may be complementary at each position in the sequence by standard base pairing or may contain one or more residues, including abasic residues, that are not complementary. Sufficiently complementary contiguous sequences typically are at least 80%, or at least 90%, complementary to a sequence to which an oligomer is intended to specifically hybridize. . Sequences that are "sufficiently complementary" allow stable hybridization of a nucleic acid oligomer with its target sequence under appropriate hybridization conditions, even if the sequences are not completely complementary. When a contiguous sequence of nucleotides of one single-stranded region is able to form a series of "canonical" or "Watson-Crick" hydrogen-bonded base pairs with an analogous sequence of nucleotides of the other single-stranded region, such that A is paired with U or T and C is paired with G, the nucleotides sequences are "completely" complementary (see. E.g., Sambrook et al., Molecular Cloning: A Laboratory Manual, 2nd ed. (Cold Spring Harbor Laboratory Press, Cold Spring Harbor, N. Y., 1989) at §§1.90-1.91, 7.37-7.57, 9.47-9.51 and 11.47-11.57, particularly §§9.50- 9.51, 11.12-11.13, 11.45-11.47 and 11.55-11.57). Appropriate hybridization conditions are well-known in the art, may be predicted based on sequence composition, or can be determined by using routine testing methods (see e.g., Sambrook et al., supra.)

As used herein, a "label" or "detection label" refers to a moiety or compound that can be detected or generate a detectable signal and that is joined directly or indirectly to a molecule, such as a probe. Direct labeling can occur through bonds or interactions that link the label to the molecule, including covalent bonds or non-covalent interactions, e.g., hydrogen bonds, hydrophobic and ionic interactions, or formation of chelates or coordination complexes. Indirect labeling can occur through use of a bridging moiety or "linker" such as a binding pair member, an antibody or additional oligomer, which is either directly or indirectly labeled, and which may amplify the detectable signal. Labels include any detectable moiety, such as a radionuclide, ligand (e.g., biotin, avidin), enzyme or enzyme substrate, reactive group, or chromophore (e.g., dye, particle, or bead that imparts detectable color), luminescent compound (e.g., bioluminescent, phosphorescent, or chemiluminescent labels), or fluorophore. Common labels used for TaqMan^{®} probes include a fluorophore and a quencher. "Fluorophore" as used herein refers to any label whose presence can be detected by its fluorescent light emitting properties. The term "quencher" as used herein refers to a moiety that absorbs at least some of the intensity of a fluorescent emission. Quenchers include fluorescent quenchers and dark quenchers (sometimes also referred to as non-fluorescent quenchers). A dark quencher is a substance that absorbs excitation energy from a fluorophore and dissipates the energy as heat, while a fluorescent quencher re-emits much of this energy as light. A fluorescent quencher is a moiety, typically a fluorophore, that can absorb the fluorescent signal emitted from a source of fluorescence at a first wavelength, for example but not limited to, a nucleic acid dye associated with a double-stranded segment of nucleic acid, and after absorbing enough fluorescent energy, the fluorescent quencher can emit fluorescence at a second wavelength that is characteristic of the quencher, a process termed "fluorescent resonance energy transfer" or FRET. Exemplary fluorophores include carboxyfluorescein (FAM^{™}), N',N'-dimethyl-N-[4-[(E)-(3-methyl-1,3-benzothiazol-2-ylidene)methyl]-1-phenylquinolin-1-ium-2-yl]-N-propylpropane-1,3-diamine (SYBR^{®} Green), ATTO fluorescent labels (Atto-Tec; Siegen, Germany), 2' -chloro-7' phenyl-1,4-dichloro-6-carboxy-fluorescein (VIC^{™}), 5'-Dichloro-dimethoxy-fluorescein dye (JOE^{™}), NED^{™}, Cyanine3 (Cy3), carboxyrhodamine (ROX^{™}), Texas red sulfonyl chloride (Texas Red), CAL Fluor^{®} Orange 560, CAL Fluor^{®} Red 610, and Cyanine5 (Cy5) dyes (all available from numerous commercial sources). Exemplary quenchers include BlackBerry^{®} Quenchers (BBQ^{™},) ATTO quenchers (Atto-Tec; Siegen, Germany), Black Hole Quenchers (BHQ^{™}), TAMRA^{™} and 4-((4-(dimethylamino)phenyl)azo)benzoic acid (DABCYL acid) (all available from numerous commercial sources). Synthesis and methods of attaching labels to nucleic acids and detecting labels are known in the art (see for example, Sambrook et al., Molecular Cloning, A Laboratory Manual, 2nd ed. (Cold Spring Harbor Laboratory Press, Cold Spring Harbor, NY, 1989), Chapter 10; U.S. Pat. Nos. 5,658,737, 5,656,207, 5,547,842, 5,283,174, and 4,581,333). More than one label, and more than one type of label, may be present on a particular probe, or detection may use a mixture of probes in which each probe is labelled with a compound that produces a different detectable signal (see, e.g., U.S. Pat. Nos. 6,180,340 and 6,350,579).

"Detection probe," "detection oligonucleotide," "detection oligomer," "probe oligomer," and "detection probe oligomer" are used interchangeably to refer to a nucleic acid oligomer that hybridizes specifically to a target sequence in a nucleic acid, such as an amplified nucleic acid, under conditions that promote hybridization to allow detection of the target sequence or amplified nucleic acid. Detection may either be direct (e.g., a probe hybridized directly to its target sequence) or indirect (e.g., a probe linked to its target via an intermediate molecular structure). Detection probes may be DNA, RNA, analogs thereof, or combinations thereof (e.g., DNA/RNA chimerics) and they may be labeled or unlabeled. Detection probes may further include alternative backbone linkages such as, e.g., 2'-O-methyl linkages. A detection probe's "target sequence" generally refers to a smaller nucleic acid sequence region within a larger nucleic acid sequence that hybridizes specifically to at least a portion of a probe oligomer by standard base pairing. A detection probe may include target-specific sequences and other sequences that contribute to the three-dimensional conformation of the probe (see, e.g., U.S. Pat. Nos. 5,118,801, 5,312,728, 6,849,412, 6,835,542, 6,534,274, and 6,361,945 and U.S. Patent Application Pub. No. 20060068417).

An "elution buffer" as used herein is a suitable liquid for separating nucleic acids from a solid support.

A "master mix buffer," "master mix," or "amplification master mix buffer" as used herein includes amplification reagents and optionally primers used to amplify a target nucleic acid but does not include the sample to be amplified.

An "amplification reaction chamber," also referred to as "reaction chamber" or "reaction space" denotes a space in which the amplification of target nucleic acids is performed. Multiple amplification reaction chambers may be arranged in parallel or substantially parallel to each other in a variety of vessels. Examples of vessels that can contain multiple amplification reaction chambers include a multi-well strip, a multi-well plate, a microfluidic chip, or a microfluidic cartridge.

A "fluorescence detector" or "fluorescence sensor" as used herein, refers to an optical detector that is capable of obtaining fluorescence measurements. Such measurements can be obtained prior to, during and after nucleic acid amplification. A fluorescence detector may be a photodiode or photomultiplier. The fluorescence detector receives light (fluorescence) emitted from a sample. Detection of PCR products in real-time can be accomplished by using fluorescent dyes or probes. The fluorescence signal, as measured by a fluorescence detector, increases at each PCR cycle as more polynucleotide molecules are generated.

As used herein, the term "relative fluorescence unit" ("RFU") is a unit of measurement of fluorescence intensity. RFU varies with the characteristics of the detection means used for the measurement and can be used as a measurement to compare relative intensities between samples and controls.

As used herein, the term "substantially" can be synonymous with the term "essentially" and indicates that a step, reagent, component, or other element achieves a result or has a property that does not materially differ, but may have minor differences or deviations, from the result or property to which "substantially" applies. For example, to "substantially avoid variation" may mean that the variation is less than or equal to about 20%, 15%, 10%, 5%, 4%, 3%, 2%, or 1%.

As used herein, the term "about" refers to a numerical value, including, for example, whole numbers, fractions, and percentages, whether or not explicitly indicated. When the term precedes a list of numerical values or ranges, the terms modify all of the values or ranges. As applied to measured quantities, the term includes the exact numerical value modified by the term, as well as ranges of values that would be expected to be within experimental error. "About" encompasses a range of plus or minus (±) 10% unless the context indicates otherwise. For example, "about 5°C" means "5°C" and a range of temperatures that is within ±10% of 5°C. The term has a similar meaning with respect to other parameters. For example, "about 5 minutes" means "5 minutes" and a range of times that is within ±10% of 5 minutes. In some contexts, the percentage of experimental error is implied or apparent and may not be explicitly stated. In some contexts, the percentage of experimental error will be provided explicitly. The term "about" may be used to modify any measurable quantity, including quantities of time, temperature, volume, mass, weight, length, density, size, percentage, ratio, dose, frequency, pressure, rate, and intensity. In some instances, the term about may include numerical values that are rounded to the nearest significant figure.

Reference to a numerical range herein (e.g., "X to Y" or "from X to Y" or "between X and Y") includes the endpoints defining the range and all values falling within the range.

The terms "a," "an," and "the" include plural referents, unless the context clearly indicates otherwise. For example, "a nucleic acid" as used herein is understood to represent one or more nucleic acids. As such, the terms "a" (or "an"), "one or more," and "at least one" can be used interchangeably herein.

"Or" is used in the inclusive sense, i.e., is equivalent to "and/or," unless the context clearly indicates otherwise.

### DETAILED DESCRIPTION

Disclosed herein are methods for monitoring a nucleic acid amplification reaction mixture for an abnormality. The methods can leverage the presence of fluorophores, quenchers (substances capable of affecting fluorescent emissions by static or dynamic quenching), and/or reagents with inherent fluorescence, e.g., dNTPs and primers, to changes in temperature and buffer composition to detect an abnormal temperature or an abnormal buffer composition in a nucleic acid amplification reaction mixture. The abnormalities may adversely affect the reliability of the data collected from the reaction. Importantly, the methods can be implemented on many existing nucleic acid amplification systems, such as real-time nucleic acid amplification systems and any other systems equipped with a fluorescence detector, without requiring additional hardware. Instead, the methods use the existing features of such systems to monitor fluorescence signals for abnormal detection during a nucleic acid amplification reaction.

### A. Detection Labels and Probes

Detection labels may be used in accordance with the present disclosure. Nucleic acid amplification reaction mixtures of the present disclosure may include detection oligomers (e.g., designed to hybridize to an amplicon) and/or amplification oligomers, such as forward and/or reverse primers, with detection labels. In general, an amplification oligomer or detection oligomer with a detection label used in an amplification reaction includes at least (1) a region for hybridizing specifically to a region on a target nucleic acid sequence and (2) a detection label. In some embodiments, an oligomer with a detection label is termed a detection probe.

Suitable fluorophores for use as detection labels can include compounds that emit a detectable light signal, e.g., fluorophores ("fluorescent dye compounds"). More than one label, and more than one type of label, may be present on a particular probe, or a mixture of probes may be used in which each probe is labeled with a compound that produces a detectable signal (see, e.g., U.S. Pat. Nos. 6,180,340 and 6,350,579). Labels may be attached to a probe by various means including covalent linkages, chelation, and ionic interactions, but preferably the label is covalently attached. Suitable fluorophores are well-known in the art and include, for example, CAL Fluor^{®} Orange 560, CAL Fluor^{®} Red 610, FAM^{™}, or ATTO 490LS. In some embodiments, the fluorophore is a temperature-sensitive fluorophore. In some embodiments, the temperature-sensitive fluorophore is sulforhodamine. In embodiments including fluorophore-labeled detection probes, each detection probe further includes a quencher. Suitable quenchers are well-known in the art and include, for example, BHQ^{™}, TAMRA, and DABCLY. In other embodiments, a detection probe includes both a fluorescent label and a quencher, a combination that is particularly useful in fluorescence resonance energy transfer (FRET) assays. Specific variations of such detection probes include, e.g., a TaqMan^{®} detection probe (see, e.g., U.S. Patent No. 5;723,591), a "molecular beacon" (see, e.g., Tyagi et al., Nature Biotechnol. 16:49-53, 1998; U.S. Patent Nos. 5,118,801 and 5,312,728), and a "molecular torch" (see, e.g., U.S. Patent Nos. 6,849,412, 6,835,542, 6,534,274 and 6,361,945). In some embodiments, the fluorophore in a nucleic acid amplification reaction mixture is associated with a probe that further includes a quencher. Probes that further include a quencher include TaqMan^{®} probes, molecular beacons and molecular torches.

### B. Reaction Mixtures

In some embodiments, a reaction mixture used in methods or systems described herein includes one or more of the amplification oligomers for amplification of a target nucleic acid. In some embodiments, the amplification oligomers include detection labels. Labeled amplification oligomers may be used to produce labeled amplicons that can be detected,'e.g., through hybridization to immobilized probes. The reaction mixture will typically include other reagents suitable for performing *in vitro* amplification such as buffers, salt solutions, appropriate nucleotide triphosphates (e.g., dATP, dCTP, dGTP, dTTP; ATP, CTP, GTP and UTP), and/or enzyme(s) (e.g., DNA polymerase, reverse transcriptase, and RNA polymerase), and may include test sample components, in which an internal control (IC) target nucleic acid may be present.

### C. Reaction Vessels; Samples

A variety of reaction vessels may be used to contain the nucleic acid amplification reaction mixtures used according to the present disclosure. Non-limiting examples of reaction vessels include tubes, multi-well strips, multi-well plates, microfluidic chips, and microfluidic cartridges. In some embodiments, a plurality of fluorophore-containing nucleic acid amplification reaction mixtures are contained in a plurality of tubes, a multi-well strip, a multi-well plate, a microfluidic chip, or a microfluidic cartridge.

In some embodiments, the reaction vessel is a microfluidic cartridge. In some embodiments the microfluidic cartridge (e.g., a "lab-on-a-chip") can perform a complete nucleic acid analysis of a sample, from sample collection to nucleic acid amplification, to the reading of the result(s). Exemplary microfluidic cartridges that can be used to perform the steps of the methods disclosed herein are depicted in Figures 7A-D. Figure 7A shows a top-down view of an exemplary microfluidic cartridge which contains multiple chambers for housing samples, reagents, or other liquids. Microchannels connect such chambers to move liquids among different functional areas within the cartridge. Figure 7B shows the bottom view of the exemplary microfluidic cartridge which shows a network of microchannels connecting various chambers to other areas within the cartridge. Figure 7C shows a bottom view of a fully constructed microfluidic cartridge which includes a sample preparation area, a nucleic acid amplification area, and a nucleic acid analysis area. These functional areas are connected by the series of microchannels shown in detail in Figure 7B. The sample preparation area includes liquids in adjacent chambers. A system, e.g., a nucleic acid amplification system, can be programmed to combine liquids contained in these chambers in discrete volumes in a particular order. When docked in the system, the nucleic acid amplification area is in close proximity to a thermal cycler, as shown in Figure 5. Similarly, when docked in a system, a nucleic acid analysis area is in close proximity to an array detector as shown in Figure 6. Figure 7D shows an exploded view of the fully constructed exemplary microfluidic cartridge which includes a cartridge body (1) a vented cap for covering the sample after input (16), a sample filter (5) and the components for the cartridge to interact with the mechanical components of the system to move and combine liquids in the sample preparations area (4, 6-8, 11). The exploded view of Figure 7D also shows how the microarray slide (10) is attached to the nucleic acid amplification area via an adhesive tape (9) in the nucleic amplification analysis area. The cartridge also includes polypropylene (PP) foil coverings (2, 3). Microfluidic cartridges can have several advantages, including the capability to conduct automated operations while consuming low reagent volumes and/or being inexpensive and disposable. Examples of microfluidic cartridges are disclosed in U.S. Patent No. 10,654,039.

In some embodiments, a microfluidic cartridge includes at least (1) a plurality of functional areas including a sample preparation area, a nucleic acid amplification area, a nucleic acid analysis area, and a waste area; (2) a central distribution hub; and (3) a pump, a plurality of valves, and a fluidic network of microchannels connecting the functional areas to the central distribution hub. An exemplary microfluidic channel is depicted in Figures 7A-7D. The pump, plurality of valves, and fluidic network of microchannels can drive movement of a fluid from a first functional area through the central distribution hub to a second functional area of the plurality of functional areas.

The sample preparation area may include of a network of reservoirs or tanks holding a plurality of liquids or gases in various configurations, as shown in Figure 7A. Such liquids or gases may contain a sample and/or reagent commonly used in the art, e.g., master mix, wash buffer, elution buffers hybridization buffers. Arrangement, distribution, and transfer of such reagents may be customized based on experimental protocols or amplification systems used.

The nucleic acid amplification area may be adjacent to the sample preparation area and connected via microfluidic channels. Such channels allow for the movement of prepared samples to the nucleic acid amplification area through the central distribution hub to be subjected to nucleic acid amplification conditions.

The nucleic acid analysis area may include a microarray component or slide, as shown in Figure 7C. A microarray slide may be used to analyze the amplified nucleic acids via capture probes. Such microarrays may include an array of many individual fragments of DNA immobilized on a solid support (e.g., glass slide) that hybridize with complementary target sequences in an organism of interest. Hybridization may be detected using a fluorescent reporter molecule, e.g., a fluorophore. The inclusion of different probe sequences on one microarray allows simultaneous detection of different organisms, or differences between organisms of the same species, allowing for syndromic testing with a high degree of specificity.

A functional area may be a space dedicated to a specific operation on the sample. The functional areas of a microfluidic cartridge may be fluidly connected to a central distribution hub by a fluidic network of microchannels. Functional areas can be arranged in a variety of ways, and multiple functional areas can be either identical to or different from each other. Examples of functional areas include a nucleic acid extraction area, a nucleic acid purification area, a nucleic acid preparation area, a nucleic acid hybridization area, a nucleic acid amplification area, a nucleic acid detection area, a nucleic acid analysis area, and a waste area. In some embodiments, the detection area is a biochip.

The central distribution hub can be connected to a pump and a plurality of valves, and the central distribution hub can pump and inject fluids from one functional area to another. Thus, the central distribution hub makes it possible to use only one simple fluid displacement system (typically a pumping system) for most of the fluid movements of the microfluidic cartridge, for inducing depressurization and pressurization in order to displace the fluid from a functional area to another one, and to reduce the volume of the microfluidic cartridge.

Each microchannel may include a central distribution hub end ("the hub end") and a functional area end ("the area end"). The area end of a microchannel is adjacent to the corresponding functional area, and the hub end is adjacent to the central distribution hub. Each microchannel may also include a valve located at or near the associated area end. Thus, the microfluidic cartridge includes a plurality of valves located at or near the area ends of the hub-connected microchannels. The plurality of valves allow for the targeted distribution of liquids and gases in channel networks, by actively opening and closing channels and by metering liquids. In some embodiments, the valves are operated in an automated manner through turning the valve in previously defined increments. In some embodiments, the valves are operated via a valve actuator.

The valves may be spatially arranged in order to be independently actuated by an actuator. In some embodiments, the actuator is an external cam-driven actuator, linear-motion actuator, a rotational-motion actuator, a linear actuator, or a rotary actuator.

In some embodiments, the fluorophore-containing nucleic acid amplification reaction mixture is located in a nucleic acid amplification area of a reaction vessel (e.g., well, chamber, etc.) during a first time period. In some embodiments, the method further includes adjusting a heating element of a nucleic acid system to an initial temperature for a predetermined time period and the fluorophore-containing nucleic acid amplification reaction mixture is located in the nucleic acid amplification area during the predetermined time period. In some embodiments, the nucleic acid amplification reaction mixture is exposed to the initial temperature for the predetermined time period to inactivate reverse transcriptase following a reverse transcription step used to transcribe RNA to DNA that can then be amplified by PCR.

As used herein, a nucleic acid amplification reaction mixture may include a sample, which may contain or be suspected of containing a target nucleic acid of interest. A variety of sample types and preparations may be used. In some embodiments, before the sample is added to a nucleic acid amplification mixture, it may be subjected to mechanical breakage, e.g., by bead beating or sonication, so that target nucleic acids may be more easily obtained from the sample. In some embodiments, extraction and purification of target nucleic acids is performed. Where a microfluidic cartridge is used, these steps may be performed in the microfluidic cartridge. In some embodiments, extraction and purification of target nucleic acids is performed in a sample preparation area of a microfluidic cartridge.

### D. Methods for Monitoring Nucleic Acid Amplification Reaction Mixtures for Abnormalities

Methods described herein can include providing a fluorophore-containing nucleic acid amplification reaction mixture. For example, the fluorophore may be provided as a fluorophore-containing nucleic acid (e.g., labeled detection probe). In some embodiments, the fluorophore-containing nucleic acid reaction mixture is a reaction mixture that is subjected to thermocycling. In some embodiments, the fluorophore-containing nucleic acid reaction mixture is a PCR reaction mixture or an RT-PCR reaction mixture.

In an aspect, the methods include (a) providing a fluorophore-containing nucleic acid amplification reaction mixture for use in a nucleic acid amplification system, and (b) adjusting a heating element of the nucleic acid amplification system to a first temperature for a first time period, thereby heating the nucleic acid amplification reaction mixture to a first incubation temperature during this, first time period. The first time period can be a high temperature step before the nucleic acid amplification reaction mixture is exposed to conditions for nucleic acid amplification (e.g., thermocycling), such as in PCR or qPCR. In some embodiments, the first time period is a reverse transcriptase inactivation period for inactivating reverse transcriptase used in RT-PCR. In some embodiments, nucleic acids in the amplification reaction mixture are denatured during the first time period. In some embodiments, the first temperature during this first time period is greater than 80°C, about 80-1 10°C, about 85-125°C, about 90-120°C, about 95-115°C, about 109°C, or about 110°C. In some embodiments, the first temperature varies during the first time period; for example, it may be higher during a first portion of the first time period than during a second portion of the first time period, in which the second portion follows the first portion. In some embodiments, the first temperature is about 95-110°C (such as 100-1 10°C, 105-1 10°C, or about 109°C) for a first portion of the first time period and about 80-105°C (such as 95-100°C or about 99°C) for a second portion of the first time period. In some embodiments, the first time period is about 20 seconds to about 10 minutes, e.g., about 2-10 minutes, about 3-9 minutes, about 3-7 minutes, or about 4-6 minutes. Where applicable, the first and second portions of the first time period may each be about half (e.g., 40-60%) of the first time period. In some embodiments, the first time period is about 48-52 seconds, which may include a delay of about 4 seconds to allow for the heating element to cross 80°C at the beginning of the first time period. In some embodiments, the first time period includes a first portion of about 20 seconds at about 109°C and a second portion of about 20 seconds at about 99°C.

In some embodiments, the first time period includes a delay of about 1 second to about 10 seconds to allow the heating element to reach or cross a target temperature for the first time period or the first portion thereof. In some embodiments, the target temperature is about 80°C. An exemplary delay of 4 seconds is shown in Figure 3. In Figure 3, the x-axis indicates the individual temperature measurements taken during the indicated amplification phases. The target temperature (target T°) indicates the programmed temperature during each phase of the reaction, whereas the heater temperature (heater T°) indicates the recorded temperature of the heating element over time. The time indicated with the arrow of 48 seconds indicates the first time period, including a delay of 4 seconds. Fluorescence signals are not recorded during such a delay.

In some embodiments, the first time period lasts about 35 seconds to about 60 seconds. In some embodiments, the first time period lasts about 5 seconds to about 50 seconds once the temperature of the first time period reaches a temperature maximum plateau. In some embodiments, the first time period lasts about 5 seconds to about 50 seconds once the temperature of the first time period crosses about 80°C.

In some embodiments, the first time period includes a delay that is sufficient to confirm that the heating element reaches or crosses a target temperature in a reaction vessel. In some embodiments, the method includes adjusting the heating element during the delay to an initial temperature for a predetermined time period. In some embodiments, the method further includes this delay before a fluorophore-containing nucleic acid amplification reaction mixture is subjected to amplification conditions. In some embodiments, the initial temperature is suitable for reverse transcription. In some embodiments, reverse transcription occurs during the predetermined time period. In some embodiments, reverse transcription occurs in a fluorophore-containing nucleic acid amplification reaction mixture during a predetermined time period. In some embodiments, the initial temperature is about 25-50°C, about 30-50°C, about 35-50°C, about 40-50°C, about 42-49°C, about 35-40°C, about 40-45°C, about 42°C, about 43°C, about 44°C, about 45°C, about 46 °C, about 47°C, about 48°C, about 50°C, or about 55°C. In some embodiments, the predetermined time period is about 15-100 minutes, e.g., about 20-50 minutes.

### 1. Fluorescence Measurements

Methods of this disclosure include measuring fluorescence from fluorophores present in the nucleic acid amplification reaction mixture. Fluorescence may be measured over the entire first time period described in the preceding section. Fluorescence may be measured during the first time period, e.g., after a delay as described in the preceding section. A first value and/or a second value may then be determined from these fluorescence measurements. Fluorescence may be measured in terms of relative fluorescence units (RFU). In some embodiments, when fluorescence is measured during the first time period, the nucleic acid amplification reaction mixture has not yet been exposed to nucleic acid amplification conditions, such as those typically found in a PCR or qPCR reaction. The fluorescence measurement described herein differs from fluorescence measurements that may be performed in quantitative and/or real-time PCR methods. This is because the fluorescence measurements in the disclosed methods are used qualitatively in that they are evaluated with respect to one or more thresholds or ranges to give a qualitative output (such as a determination of whether there is an abnormality, depending on whether the fluorescence measurements satisfy the threshold or are within the predetermined range). Fluorescence measurements that fail to satisfy the threshold or fail to fall within the predetermined range may be indicative of a defective heating element, a defective thermal contact, a defective thermocycler, a defective fluorescence detector, insufficient or incorrect reagents within the reaction mixtures, and/or other abnormalities. Furthermore, the measurements may be made during a period before thermocycling commences, and the present methods do not require fluorescence measurements during thermocycling to detect abnormalities.

In some embodiments, the step of providing a fluorophore-containing nucleic acid amplification reaction mixture for use in a nucleic acid amplification system includes providing a plurality of fluorophore-containing nucleic acid amplification reaction mixtures. This plurality of fluorophore-containing nucleic acid amplification reaction mixtures may be provided in a reaction vessel that allows for multiple reactions to be run in parallel, while contained in a single vessel. Such vessels include multi-well strips, multi-well plates, microfluidic chips, and microfluidic cartridges. In some embodiments, the step measuring fluorescence from the fluorophore at a plurality of times during the first time period includes measuring fluorescence from the fluorophores of each of the plurality of fluorophore-containing nucleic acid amplification reaction mixtures contained in the plurality of vessels (e.g., tubes, multi-well strips, multi-well plates, microfluidic chips, or microfluidic cartridges).

In some embodiments, a single value is determined from the fluorescence measurements. In other embodiments, both a first value and a second value are determined from the fluorescence measurements.

In some embodiments, a first value, which represents a change in fluorescence signal during the first time period, is determined for each of the plurality of fluorophore-containing nucleic acid amplification reaction mixtures. In some embodiments, a second value, which represents a rate of change in fluorescence during the first time period, is determined for each of the plurality of fluorophore-containing nucleic acid amplification reaction mixtures. In some embodiments, the first and second values are determined for each of the plurality of fluorophore-containing nucleic acid amplification reaction mixtures.

### a) Determination of a First Value from Fluorescence Measurements

In some embodiments, the first value is a measure of the total change in fluorescence during the first time period. In some embodiments, the first value is determined from a plurality of fluorescence measurements which are recorded over time. If one presumes that the fluorescence of a fluorophore increases in response to rising temperature, then the change in fluorescence is the total change in fluorescence from the beginning of the first time period, when the temperature of the fluorophore-containing nucleic acid amplification reaction mixture is at its lowest, to the end of the first time period, when the temperature of the fluorophore-containing nucleic acid amplification reaction mixture is at its highest. Thus, in some embodiments, the change in fluorescence during this first time period is the total increase or the total gain in fluorescence. In some embodiments, the first fluorescence measurement is the lowest fluorescence measurement during the first time period. In some embodiments, the last fluorescence measurement is recorded at the end of the first time period, after a maximum temperature has been maintained by the heating element for a period of time and the fluorescent signal has plateaued. In these embodiments, the last fluorescence measurement may be the highest fluorescence measurement during the first time period.

In some embodiments, fluorescence measurements are recorded at fixed time intervals. In some embodiments, a fluorescence measurement is recorded about every 100 milliseconds, about every 200 milliseconds, about every 300 milliseconds, about every 400 milliseconds, or about every 500 milliseconds. In some embodiments, fluorescence measurements are recorded over a period of time, such as about 1 second to about 10 seconds.

In some embodiments, the first fluorescence measurement is recorded at the beginning of the first time period when the temperature of the heating element has arrived at a target temperature for the first time period. In some embodiments, the first fluorescence measurement is recorded after the heating element arrives at 80°C or crosses 80°C.

In some embodiments, a first measurement is obtained at about 1 second to about 10 seconds before the first time period begins. In some embodiments, a first measurement is obtained at about 1 second to about 10 seconds before the heating element arrives at 80°C or crosses 80°C. In some embodiments, the first measurement is obtained at about 1 second to about 10 seconds after the first time period begins. In some embodiments, the first measurement is obtained at about 1 second to about 10 seconds after the heating element arrives at 80°C or crosses 80°C.

In some embodiments, the last measurement is obtained at about 35 seconds to about 60 seconds after the start of the first time period. In some embodiments, the last measurement is obtained at about 5 seconds to about 30 seconds after the temperature during the first time period reaches a temperature plateau.

In some embodiments, the first value represents a change in fluorescence signal measured in relative fluorescent units. In some embodiments, the first value is determined by subtracting an earlier fluorescence measurement from a later fluorescence measurement. In some embodiments, the earlier fluorescence measurement is obtained before or during the first half of the first time period, within one minute of the beginning of the first time period, or at about the beginning of the first time period. In some embodiments, the earlier fluorescence measurement constitutes the least measured fluorescence from the fluorophore. In some embodiments, the later fluorescence measurement is obtained during the second half of the first time period, within one minute of the end of the first time period, or at about the end of the first time period. In some embodiments, the later fluorescence measurement constitutes the greatest measured fluorescence from the fluorophore. In some embodiments, the later fluorescence measurement in the first time period minus the earlier fluorescence measurement in the first time period is termed the "contrast."

### b) Determination of a Second Value from Fluorescence measurements

In some embodiments, the second value is a thermal ratio that is equal to a lower area of a quadrilateral region divided by an upper area of the same quadrilateral region. An exemplary quadrilateral region and the upper and lower areas thereof are shown in Fig. 1. During the first time period described above, a plot with a quadrilateral region, e.g., a graph, may be produced to represent the change (i.e., gain) in fluorescence over time. For example, units of time may be shown on an x-axis and relative fluorescence units (RFU) may be shown on a y-axis, as illustrated in Fig. 1. As such, in some embodiments, fluorescence signals can be plotted over time to generate a curve. In some embodiments, the quadrilateral region is bounded vertically by the earlier and later fluorescence measurements from the fluorophore during the first time period and horizontally by the times of the earlier and later fluorescence measurements from the fluorophore during the first time period. In some embodiments, the quadrilateral region is bounded vertically by the lowest and highest fluorescence measurements from the fluorophore during the first time period and horizontally the times of the lowest and highest fluorescence measurements from the fluorophore during the first time period. In some embodiments, the quadrilateral region is bounded vertically by the lowest and highest fluorescence measurements from the fluorophore during the first time period and horizontally by the times of the earlier and later fluorescence measurements from the fluorophore during the first time period. In some embodiments, the quadrilateral region is bounded vertically by the earlier and later fluorescence measurements from the fluorophore during the first time period and horizontally by the times of the lowest and highest fluorescence measurements from the fluorophore during the first time period.

In some embodiments, the plot includes a curve that represents a change in fluorescence over time. In some embodiments, the plot includes a histogram, from which a curve may be produced by drawing a line through the RFU data points of each bar in the histogram and a median filter is applied to smooth out the curve. In either embodiment, the curve represents a boundary between (1) a lower area of the quadrilateral region and (2) an upper area of the quadrilateral region. Therefore, (1) the lower area can equal the area of the quadrilateral region below the fluorescence measurements from the fluorophore, and (2) the upper area can equal the area of the quadrilateral region above the fluorescence measurements from the fluorophore. Thus, the surface areas (or two-dimensional spaces) of the lower area and the upper area may be defined by the curve and the boundaries of the quadrilateral region.

The shape of the curve during the first time period described above may be quantified by determining the ratio of the lower area to the upper area. This surface ratio of the lower area to the upper area may represent the second value. In some embodiments, the lower area is termed the first subvalue and the upper area is termed the second subvalue. Accordingly, the second value can be determined by dividing the first subvalue by the second subvalue. In some embodiments, the first subvalue represents a lower area of a quadrilateral region of a plot of fluorescence from the fluorophore versus time during the first time period. In some embodiments, the second subvalue represents an upper area of the quadrilateral region. In some embodiments, the second value represents the rate of change in fluorescence over time, and the rate of change correlates with a rate of temperature change in the nucleic acid amplification reaction mixture.

In some embodiments, fluorescence measurements are recorded at fixed time intervals. In some embodiments, a curve-smoothing function is applied to the measurements recorded at fixed time intervals. In these embodiments, the exact areas above the curve and below the curve are not determined. In these embodiments, the lower area, i.e., the first subvalue, is the sum of a series of differences between the minimum values, which are represented by the data points along the curve. Also in these embodiments, the upper area, i.e., the second subvalue, is the sum of the maximum value from the quadrilateral region minus each minimum value.

### c) Determination of Abnormalities by Comparison of First and Second Values with Predetermined Values

In some embodiments, the first and second values, e.g., (1) the gain in RFU and (2) the surface ratio of the lower area to the upper area (first subvalue/second subvalue), are used to identify nucleic acid amplification reaction mixtures associated with an abnormality. These first and second values may be determined for a nucleic acid amplification reaction mixture of interest. The first and second values may then be compared to first and second values determined from reference nucleic acid amplification reaction mixtures.

In some embodiments, in the step of comparing the first and/or second values to the first and/or second predetermined threshold or the first and/or second predetermined range, for each of the plurality of fluorophore-containing nucleic acid amplification reaction mixtures, (1) the first value is compared to a first predetermined threshold or range, (2) the second value is compared to a second predetermined threshold or range, or (3) the first value is compared to a first predetermined threshold or range, and the second value is compared to a second predetermined threshold.

Reference runs of nucleic acid amplification reactions may include a plurality of successful and unsuccessful reactions. The reference runs can include reactions that were performed under different conditions, ranging from normal to completely defective due to bad thermal contact, a defective thermocycler; or fluidic anomalies, including an insufficient amount of reagents. The unsuccessful reactions can include nucleic acid amplification reaction mixtures with one or more conditions that caused those reactions to fail. The first and second values for each of these reference reactions may be determined, and in some embodiments, the first and second values are termed the first predetermined value and the second predetermined value. In some embodiments, the first predetermined value is a range or a threshold. In some embodiments, the second predetermined value is a range or a threshold.

### 2. Abnormalities

To detect abnormalities in a nucleic acid amplification reaction mixture, in some embodiments, a first value of the methods described herein is compared to a first predetermined threshold or range. In some embodiments, a second value of the methods described herein is compared to a second predetermined threshold or range. These comparisons allow for the detection of an abnormal curve shape. In some embodiments, in the event that an abnormality is detected, the nucleic acid amplification system provides an alarm. Advantageously, the present methods and systems do not require additional reagents relative to existing nucleic acid amplification reactions. Also advantageously, the present methods can be implemented on existing nucleic acid amplification systems that are capable of detecting fluorescence without alteration of the hardware thereof. The present methods and systems collect and use fluorescence that is commonly generated but disregarded in existing methods. Such fluorescence is used in a novel manner to detect abnormalities, and as such can improve assay throughput and efficiency (e.g., by enabling early termination of assays with abnormalities) without requiring additional hardware, reagents, or space.

In some embodiments, an abnormality is detected in a nucleic acid amplification reaction mixture when the first value indicates a change in fluorescence that does not satisfy the first predetermined threshold or that is outside the first predetermined range.

In some embodiments, an abnormality is detected in a nucleic acid amplification reaction mixture when the second value indicates a rate of temperature change that does not satisfy the second predetermined threshold or that is outside the second predetermined range.

In some embodiments, an abnormality is detected in a nucleic acid amplification reaction mixture when either (1) the first value indicates a change in fluorescence that does not satisfy the first predetermined threshold or that is outside the first predetermined range, or (2) the second value indicates a rate of temperature change that does not satisfy the second predetermined threshold or that is outside the second predetermined range.

In some embodiments, an abnormality is detected in a nucleic acid amplification reaction mixture when (1) the first value indicates a change in fluorescence that does not satisfy the first predetermined threshold or that is outside the first predetermined range, and (2) the second value indicates a rate of temperature change that does not satisfy the second predetermined threshold or that is outside the second predetermined range.

In some embodiments, an abnormality is detected when the sum of the maximum value from the quadrilateral region minus each minimum value (i.e., the second subvalue) is greater than a predetermined limit or threshold for that sum. In some embodiments, an abnormality is detected with the second value (i.e., the thermal ratio) is greater than a predetermined limit or threshold for the second value.

In some embodiments, the abnormality includes defective thermal contact. For example, there can be poor or inadequate contact between the heating element in a nucleic acid amplification system and a reaction vessel containing a nucleic acid amplification reaction mixture. Defective thermal contact can lead to improper heat transfer to the nucleic acid amplification reaction mixture, thereby causing an abnormality in the nucleic acid amplification reaction.

In some embodiments, the abnormality includes a defective heating element. In some embodiments, the defective heating element is defective in controlling or changing temperature or in transferring heat to the nucleic acid amplification reaction chamber.

In some embodiments, the abnormality includes defective liquid handling. In some instances, there can be errors in liquid handling during preparation of the nucleic acid amplification reaction mixture. Bubbles, inaccurate liquid volumes, and/or incorrect liquid reagents (such as buffers, salts, and other aqueous solutions) can be introduced to a reaction vessel, thereby causing an abnormality in the nucleic acid amplification reaction. Inaccurate liquid volumes can include insufficient liquid volume, excessive liquid volume and/or no liquid volume transferred to a nucleic acid amplification reaction chamber. Incorrect liquid reagents can include nucleic acid amplification reaction mixtures with one or more reagent changes that cause a reaction to fail.

### E. Nucleic Acid Amplification System

Disclosed herein are nucleic acid amplification systems. A nucleic acid amplification system refers to a device or an apparatus that may be used to perform, monitor, and/or analyze nucleic acid amplification reactions described herein. Nucleic acid amplification systems may include a docking station configured to receive a reaction vessel for containing at least one nucleic acid amplification mixture; a heating element disposed in proximity to a position to be occupied by a nucleic acid amplification mixture when the reaction vessel is present in the docking station; a fluorescence detector configured to measure fluorescence of a fluorophore in the nucleic acid amplification mixture; and a processor operably linked to the heating element and a memory; the memory comprising instructions that when executed by the processor cause the nucleic acid amplification system to perform a method of monitoring a nucleic acid amplification reaction mixture including a fluorophore for an abnormality. The method of monitoring includes adjusting a heating element of the nucleic acid amplification system to a first temperature for a first time period; after which measuring fluorescence with a fluorescence detector of the nucleic acid amplification system from a fluorophore contained in a nucleic acid amplification mixture at a plurality of times during the first time period. The first time period may occur before a scheduled nucleic acid amplification. The method then includes determining a first value representative of a change in fluorescence signal during the first time period; and/or a second value representative of the rate of change in fluorescence during the first time period; and comparing the first value to a first predetermined threshold or range, and/or comparing the second value to a second predetermined threshold or range. An abnormality is detected if the first value indicates a change in fluorescence that does not satisfy the first predetermined threshold or that is outside the first predetermined range; the second value indicates a rate of temperature change that does not satisfy the second predetermined threshold or that is outside the second predetermined range; either the first value indicates a change in fluorescence that does not satisfy the first predetermined threshold or that is outside the first predetermined range, or the second value indicates a rate of temperature change that does not satisfy the second predetermined threshold or that is outside the second predetermined range; or the first value indicates a change in fluorescence that does not satisfy the first predetermined threshold or that is outside the first predetermined range and the second value indicates a rate of temperature change that does not satisfy the second predetermined threshold or that is outside the second predetermined range.

In some embodiments, the nucleic acid amplification system includes a docking station to receive one or more vessels. In some embodiments, the vessel is a multi-chambered receptacle or the nucleic acid amplification chamber is contained within a multi-chambered receptacle. In some embodiments, when a reaction vessel is present in the system, e.g., in a docking station, the heating element is disposed in proximity to a position occupied by or to be occupied by a nucleic acid amplification reaction mixture (i.e., a functional area of the reaction vessel in thermal communication with the heating element).

In some embodiments, the nucleic acid amplification system includes a temperature controller and is capable of providing or transferring heat to nucleic acid amplification reaction chambers via one or more heating elements. In some embodiments, the nucleic acid amplification system is programmable such that it can hold nucleic acid amplification reaction chambers at set temperatures for different lengths of time.

In some embodiments, the nucleic acid amplification system includes a optical detector (e.g., fluorometer) configured to measure and record the fluorescence of one or more fluorophores in nucleic acid amplification reaction mixtures. Fluorescence measurements and recordings may be single events or multiple events over time.

In some embodiments, the nucleic acid amplification system may also include sensors to monitor and/or estimate temperature or liquid filling of the nucleic acid amplification reaction mixtures. In some embodiments, a sensor may be a thermal sensor, a capacitive sensor, or an infrared sensor. The nucleic acid amplification system may include a processor operably linked to the heating element and a memory. The memory includes instructions that can cause the system to perform methods of monitoring a nucleic acid amplification reaction mixture for an abnormality described herein.

In some embodiments, in the event that an abnormality is detected, the nucleic acid amplification system provides one or more alarms. Examples of alarms include visual notifications and audio notifications. In some examples, when an alarm is provided, the nucleic acid amplification system pauses a method of nucleic acid amplification, which may include adjusting the heating element to about 4°C and holding that temperature for a period of time.

In some embodiments, the system is configured to detect an abnormality including a defective heater (e.g., thermal cycler), inadequate contact between the heater and a reaction vessel (e.g., microfluidic cartridge), inadequate passage of a reaction reagent or nucleic acid amplification reaction mixture into the functional area for performing a nucleic acid amplification reaction (e.g., PCR), or an issue with the composition of a reaction mixture.

In some embodiments, the system is configured to adjust the heating element to an initial temperature for a predetermined time period before providing a fluorophore-containing nucleic acid amplification reaction mixture in a nucleic acid amplification system. The initial temperature may be suitable for reverse transcription. Reverse transcription may occur in the fluorophore-containing nucleic acid amplification reaction mixture during the predetermined time period, as described herein.

In some embodiments, the system is configured to subject the nucleic acid amplification reaction mixture to conditions for performing themocycling, provided an abnormality is not detected. Such conditions may include time and temperature conditions for performing PCR or RT-PCR.

The fluorophore-containing nucleic acid amplification reaction mixture may be contained in a microfluidic cartridge, which may have any of the features described elsewhere herein with respect to such cartridges.

In some embodiments, the system is configured to receive a plurality of fluorophore-containing nucleic acid amplification reaction mixtures contained in a multi-well plate or in a plurality of tubes. For example, the system may include a receptacle for a multi-well plate or a plurality of receptacles for tubes.

In some embodiments, the system is configured to detect an abnormality if the first value indicates a change in fluorescence that does not satisfy the first predetermined threshold or that is outside the first predetermined range. In some embodiments, the system is configured to detect an abnormality if the second value indicates a rate of temperature change that does not satisfy the second predetermined threshold or that is outside the second predetermined range. In some embodiments, the system is configured to detect an abnormality if either the first value indicates a change in fluorescence that does not satisfy the first predetermined threshold or that is outside the first predetermined range or the second value indicates a rate of temperature change that does not satisfy the second predetermined threshold or that is outside the second predetermined range. In some embodiments, the system is configured to detect an abnormality if the first value indicates a change in fluorescence that does not satisfy the first predetermined threshold or that is outside the first predetermined range and the second value indicates a rate of temperature change that does not satisfy the second predetermined threshold or that is outside the second predetermined range.

In some embodiments, the system is configured such that adjusting the heating element to the first temperature for the first time period is a reverse transcriptase inactivation step.

In some embodiments, the method includes subjecting the nucleic acid amplification reaction mixture to nucleic acid amplification conditions only if an abnormality is not detected. In some embodiments, the nucleic acid amplification conditions include thermocycling. In some embodiments, the method includes discontinuing a nucleic acid amplification reaction if an abnormality is detected. In some embodiments, the system is configured to subject the nucleic acid amplification reaction mixture to nucleic acid amplification conditions only if an abnormality is not detected. In some embodiments, the system is configured to reduce the temperature of the heating element (e.g., to ambient temperature or a temperature greater than 0°C and less than ambient temperature, such as 0.1-10°C) without thermocycling if an abnormality is detected.

Also disclosed herein are computer-readable media. In some embodiments, a computer-readable medium includes instructions that when executed by a processor of a nucleic acid amplification system, cause the nucleic acid amplification system to perform a method of monitoring a nucleic acid amplification reaction mixture described herein. The method of monitoring includes adjusting a heating element of the nucleic acid amplification system to a first temperature for a first time period; after which measuring fluorescence with a fluorescence detector of the nucleic acid amplification system from a fluorophore contained in a nucleic acid amplification mixture at a plurality of times during the first time period. The first time period may occur before a scheduled nucleic acid amplification. The method then includes determining a first value representative of a change in fluorescence signal during the first time period; and/or a second value representative of the rate of change in fluorescence during the first time period; and comparing the first value to a first predetermined threshold or range, and/or comparing the second value to a second predetermined threshold or range. An abnormality is detected if the first value indicates a change in fluorescence that does not satisfy the first predetermined threshold or that is outside the first predetermined range; the second value indicates a rate of temperature change that does not satisfy the second predetermined threshold or that is outside the second predetermined range; either the first value indicates a change in fluorescence that does not satisfy the first predetermined threshold or that is outside the first predetermined range, or the second value indicates a rate of temperature change that does not satisfy the second predetermined threshold or that is outside the second predetermined range; or the first value indicates a change in fluorescence that does not satisfy the first predetermined threshold or that is outside the first predetermined range and the second value indicates a rate of temperature change that does not satisfy the second predetermined threshold or that is outside the second predetermined range.

In some embodiments, the computer-readable medium includes instructions for performing methods of monitoring a nucleic acid amplification reaction mixture as described herein. In some embodiments, these instructions are executed by a processor of the nucleic acid amplification system as described herein.

### EXAMPLES

### Example 1

This Example describes performing reference runs of nucleic acid amplification reactions under normal conditions and under conditions with anomalies. This collection of reference runs provides the basis for determining signal gains and thermal ratios for normal runs and for runs with anomalies. The objective is to implement additional controls into nucleic acid amplification reaction runs to detect from the run data any thermal abnormalities before completion of the reaction runs.

Reaction mixtures including Amplidiag^{®} Multiplex PCR Master Mix; a nucleic acid template; forward and reverse primers; and fluorescent probes were prepared: The first chamber contained 0.6µM ATTO with quencher (LSS signal), 0.4µM 6-FAM with quencher (blue signal), and 0.4µM Quasar^{®} 670 with quencher (red signal). The second chamber contained 1.2µM ATTO with quencher (LSS signal), 0.4µM 6-FAM with quencher (blue signal) and 0.8µM Quasar^{®} 670 with quencher (red signal).

The reaction mixtures were subjected to a protocol including the following steps, which was performed using a Novodiag^{®} system (Mobodiag Oy; Espoo, Finland):

| | |
|---|---|
| 46°C | 300 seconds |
| 109°C | 24 seconds |
| 99°C | 24 seconds |

Reactions with anomalies included reactions with bad thermal contacts and reactions with fluidic errors. Thermal errors were created by adding a layer of adhesive between the heater and the qPCR chamber, which interfered with heat transfer. Fluidic errors were created by altering the protocol to miss one or several injection steps. Additional fluidic errors were made by changing valve forces and/or positions to create leakage or prevent the valves from opening.

The system began recording RFUs after the heating element crossed 80°C plus a four second delay. The system stopped recording RFUs at the end of the 109°C period. In this example, data points were recorded every 200 milliseconds over a total of 48 seconds.

For each run, a curve of the type shown in Figure 1 was produced; a curve-smoothing function with a mean-filtering window of 5 points was applied. Then, the minimum and maximum values for each smoothed curve were also determined and signal gains were calculated as the difference thereof. Next, the thermal ratios (i.e., upper area/lower area) from each smoothed curve were determined.

**Results:** The signal gains and thermal ratios for a plurality of runs are plotted in Figure 2. A distribution of normal runs is indicated and bound by an upper and lower limit for thermal ratio, and by an upper and lower limit for signal gain. Most runs with fluidic errors have signal gains and/or thermal ratios that are higher than the upper limits for normal runs. Most runs with bad thermal contacts have thermal ratios that are lower than the lower limit for normal runs. Thus, the method was effective at detecting both fluidic errors and bad thermal contacts based on fluorescence data obtained during heating and incubation of a nucleic acid reaction mixture, such as an initial denaturation step in a thermocycled amplification reaction.

### EQUIVALENTS

The foregoing written specification is considered to be sufficient to enable one skilled in the art to practice the embodiments. The foregoing description and Examples detail certain embodiments and describe the best mode contemplated by the inventors. It will be appreciated, however, that no matter how detailed the foregoing may appear in text, the embodiment may be practiced in many ways and should be construed in accordance with the appended claims and any equivalents thereof.

## Claims

1. A method of monitoring a nucleic acid amplification reaction mixture for an abnormality, the method comprising:
a) providing a fluorophore-containing nucleic acid amplification reaction mixture in a nucleic acid amplification system;
b) adjusting a heating element of the nucleic acid amplification system to a first temperature for a first time period, thereby heating the nucleic acid amplification reaction mixture to a first incubation temperature during the first period;
c) after step b), measuring fluorescence from the fluorophore at a plurality of times during the first time period, wherein the nucleic acid amplification reaction mixture is not exposed to nucleic acid amplification conditions prior to or during the first time period;
d) determining:
i) a first value representative of a change in fluorescence signal during the first time period; and/or
ii) a second value representative of the rate of change in fluorescence during the first time period; and
e) comparing the first value to a first predetermined threshold or range, and/or comparing the second value to a second predetermined threshold or range, wherein an abnormality is detected if
i) the first value indicates a change in fluorescence that does not satisfy the first predetermined threshold or that is outside the first predetermined range;
ii) the second value indicates a rate of temperature change that does not satisfy the second predetermined threshold or that is outside the second predetermined range;
iii) either the first value indicates a change in fluorescence that does not satisfy the first predetermined threshold or that is outside the first predetermined range, or the second value indicates a rate of temperature change that does not satisfy the second predetermined threshold or that is outside the second predetermined range; or
iv) the first value indicates a change in fluorescence that does not satisfy the first predetermined threshold or that is outside the first predetermined range, and the second value indicates a rate of temperature change that does not satisfy the second predetermined threshold or that is outside the second predetermined range.

2. The methodof claim 1, wherein the first value is determined by subtracting an earlier measurement of fluorescence from the fluorophore from a later measurement of fluorescence from the fluorophore optionally wherein:
a) the earlier measurement of fluorescence from the fluorophore is obtained before or during the first half of the first time period, within one minute of the beginning of the first time period, or at about the beginning of the first time period and/or
b) the earlier measurement of fluorescence from the fluorophore is the lowest measurement of fluorescence from the fluorophore.

3. The method of claim 2, wherein the later measurement of fluorescence from the fluorophore is obtained during the second half of the first time period, within one minute of the end of the first time period, or at about the end of the first time period, optionally wherein the later measurement of fluorescence from the fluorophore is the highest measurement of fluorescence from the fluorophore.

4. The method of claim 1 or 2, wherein
a) the second value is determined by dividing a first subvalue by a second subvalue;
b) the first subvalue represents a lower area of a quadrilateral region of a plot of fluorescence from the fluorophore versus time during the first time period; and
c) the second subvalue represents an upper area of the quadrilateral region, the quadrilateral region being bounded by
i) first and last measurements of fluorescence from the fluorophore during the first time period and the times of the first and last measurements of fluorescence from the fluorophore during the first time period;
ii) minimum and maximum measurements of fluorescence from the fluorophore during the first time period and the times of the minimum and maximum measurements of fluorescence from the fluorophore during the first time period;
iii) minimum and maximum measurements of fluorescence from the fluorophore during the first time period and the times of first and last measurements of fluorescence from the fluorophore during the first time period; or
iv) first and last measurements of fluorescence from the fluorophore during the first time period and the times of minimum and maximum measurements of fluorescence from the fluorophore during the first time period;
wherein the lower area equals the area of the quadrilateral region below the measurements of fluorescence from the fluorophore and the upper area equals the area of the quadrilateral region above the measurements of fluorescence from the fluorophore.

5. The method of any one of the preceding claims, wherein the first value represents Y-axis change in fluorescence signal.

6. The method of any one of the preceding claims, wherein the fluorescence changes over time during the first time period, and the rate of fluorescence change during the first time period correlates with a rate of temperature change in the nucleic acid amplification reaction mixture.

7. The method of any one of the preceding claims, wherein nucleic acid in the fluorophore-containing nucleic acid amplification reaction mixture is denatured during the first time period, optionally wherein the first time period is in the range of about 2-10 minutes, 3-9 minutes, 3-7 minutes, or 4-6 minutes, or is about 5 minutes.

8. The method of any one of the preceding claims, wherein the first temperature is in the range of 90°C-120°C or 95°C-115°C, or is about 110°C, optionally wherein the preliminary temperature is suitable for reverse transcription, and optionally wherein reverse transcription occurs in the fluorophore-containing nucleic acid amplification reaction mixture during the preliminary time period.

9. The method of any one of the preceding claims, wherein the method further comprises adjusting the heating element to a preliminary temperature for a preliminary time period before step a), optionally wherein the preliminary temperature is in the range of 25°C to 50°C, 30°C to 50°C, 35°C to 50°C, 40°C to 50°C, or 42°C to 49°C, or is about 48°C, and optionally wherein the preliminary time period is in the range of 15-60 minutes, 20-50 minutes, or 25-40 minutes, or is about 30 minutes,

10. The method of any one of the preceding claims, wherein the fluorophore-containing nucleic acid amplification reaction mixture is a thermocycled reaction mixture, a PCR reaction mixture or an RT-PCR reaction mixture, and optionally wherein the reaction mixture is contained in a microfluidic cartridge.

11. The method of claim 10, wherein the microfluidic cartridge comprises:
a) a plurality of functional areas comprising a sample preparation area, a nucleic acid amplification area, and a waste area, optionally wherein the fluorophore-containing nucleic acid amplification reaction mixture is located in a nucleic acid amplification area during the first time period;
b) a central distribution hub; and
c) a pump, a plurality of valves, and a fluidic network of microchannels connecting the functional areas to the hub;
wherein the pump, plurality of valves, and fluidic network of microchannels can drive movement of a fluid from a first functional area through the central distribution hub to a second functional area of the plurality of functional areas, optionally wherein the method further comprises adjusting the heating element to a preliminary temperature for a preliminary time period before step a) and the fluorophore-containing nucleic acid amplification reaction mixture is located in the nucleic acid amplification area during the preliminary time period.

12. The method of any one of the preceding claims, wherein a plurality of fluorophore-containing nucleic acid amplification reaction mixtures is provided in the nucleic acid amplification system in step a), optionally wherein:
a) fluorescence is measured from the fluorophores of each of the plurality of fluorophore-containing nucleic acid amplification reaction mixtures in step c);
b) the first value representative of a change in fluorescence signal during the first time period, and/or the second value representative of the rate of change in fluorescence during the first time period, is determined for each of the plurality of fluorophore-containing nucleic acid amplification reaction mixtures in step d), optionally wherein for each of the plurality of fluorophore-containing nucleic acid amplification reaction mixtures, the first value is compared to a first predetermined threshold or range and/or the second value is compared to a second predetermined threshold or range in step e); and/or
c) the plurality of fluorophore-containing nucleic acid amplification reaction mixtures is contained in a multi-well plate or in a plurality of tubes.

13. The method of any one of the preceding claims, wherein an abnormality is detected:
a) if the first value indicates a change in fluorescence that does not satisfy the first predetermined threshold or that is outside the first predetermined range;
b) if the second value indicates a rate of temperature change that does not satisfy the second predetermined threshold or that is outside the second predetermined range;
c) if either the first value indicates a change in fluorescence that does not satisfy the first predetermined threshold or that is outside the first predetermined range or the second value indicates a rate of temperature change that does not satisfy the second predetermined threshold or that is outside the second predetermined range; or
d) if the first value indicates a change in fluorescence that does not satisfy the first predetermined threshold or that is outside the first predetermined range and/or the second value indicates a rate of temperature change that does not satisfy the second predetermined threshold or that is outside the second predetermined range; and optionally wherein the abnormality is defective thermal contact and/or defective liquid handling.

14. The method of any one of the preceding claims, wherein:
a) the fluorophore is associated with an oligonucleotide probe, optionally wherein the oligonucleotide probe further comprises a quencher;
b) the adjusting the heating element to the first temperature for the first time period is a reverse transcriptase inactivation step; and/or
c) the fluorophore is temperature-sensitive, optionally wherein the fluorophore is sulforhodamine.

15. The method of any one of the preceding claims, wherein an abnormality is detected and the nucleic acid amplification reaction mixture is not subjected to nucleic acid amplification conditions, or wherein no abnormality is detected and the nucleic acid amplification reaction mixture is subjected to nucleic acid amplification conditions.

16. The method of any one of the preceding claims, wherein the method further comprises subjecting the nucleic acid amplification reaction mixture to nucleic acid amplification conditions only if an abnormality is not detected, or wherein the method further comprises reducing the temperature of the heating element without thermocycling if an abnormality is detected.

17. A nucleic acid amplification system comprising:
a docking station configured to receive a reaction vessel for containing at least one nucleic acid amplification mixture;
a heating element disposed in proximity to a position to be occupied by a nucleic acid amplification mixture when the reaction vessel is present in the docking station;
a fluorescence detector configured to measure fluorescence of a fluorophore in the nucleic acid mixture;
and a processor operably linked to the heating element and a memory;
the memory comprising instructions that when executed by the processor cause the nucleic acid amplification system to perform a method of monitoring a nucleic acid amplification reaction mixture comprising a fluorophore for an abnormality, the method comprising:
a) adjusting the heating element to a first temperature for a first time period;
b) after step a), measuring fluorescence with the fluorescence detector from a fluorophore contained in the nucleic acid amplification mixture at a plurality of times during the first time period, wherein the first time period occurs before a scheduled nucleic acid amplification;
c) determining
i) a first value representative of a change in fluorescence signal during the first time period; and/or
ii) a second value representative of the rate of change in fluorescence during the first time period; and
d) comparing the first value to a first predetermined threshold or range, and/or comparing the second value to a second predetermined threshold or range, wherein an abnormality is detected if
i) the first value indicates a change in fluorescence that does not satisfy the first predetermined threshold or that is outside the first predetermined range;
ii) the second value indicates a rate of temperature change that does not satisfy the second predetermined threshold or that is outside the second predetermined range;
iii) either the first value indicates a change in fluorescence that does not satisfy the first predetermined threshold or that is outside the first predetermined range, or the second value indicates a rate of temperature change that does not satisfy the second predetermined threshold or that is outside the second predetermined range; or
iv) the first value indicates a change in fluorescence that does not satisfy the first predetermined threshold or that is outside the first predetermined range and the second value indicates a rate of temperature change that does not satisfy the second predetermined threshold or that is outside the second predetermined range.

18. The system of claim 16, wherein the system is configured not to subject the nucleic acid amplification reaction mixture to nucleic acid amplification conditions if an abnormality is detected and/or is configured to subject the nucleic acid amplification reaction mixture to nucleic acid amplification conditions only if an abnormality is not detected.

19. The system of claim 16 or 17, wherein the memory comprises instructions that when executed by the processor cause the nucleic acid amplification system to perform the method according to any one of claims 1 to 16.

20. A computer-readable medium comprising:
instructions that when executed by a processor of a nucleic acid amplification system cause the nucleic acid amplification system to perform a method of monitoring a nucleic acid amplification reaction mixture comprising a fluorophore for an abnormality, the method comprising:
a) adjusting a heating element of the nucleic acid amplification system to a first temperature for a first time period;
b) after step a), measuring fluorescence with a fluorescence detector of the nucleic acid amplification system from a fluorophore contained in a nucleic acid amplification mixture at a plurality of times during the first time period, wherein the first time period occurs before a scheduled nucleic acid amplification;
c) determining
i) a first value representative of a change in fluorescence signal during the first time period; and/or
ii) a second value representative of the rate of change in fluorescence during the first time period; and
d) comparing the first value to a first predetermined threshold or range, and/or comparing the second value to a second predetermined threshold or range, wherein an abnormality is detected if
i) the first value indicates a change in fluorescence that does not satisfy the first predetermined threshold or that is outside the first predetermined range;
ii) the second value indicates a rate of temperature change that does not satisfy the second predetermined threshold or that is outside the second predetermined range;
iii) either the first value indicates a change in fluorescence that does not satisfy the first predetermined threshold or that is outside the first predetermined range, or the second value indicates a rate of temperature change that does not satisfy the second predetermined threshold or that is outside the second predetermined range; or
iv) the first value indicates a change in fluorescence that does not satisfy the first predetermined threshold or that is outside the first predetermined range and the second value indicates a rate of temperature change that does not satisfy the second predetermined threshold or that is outside the second predetermined range.

21. The computer-readable medium of claim 20, comprising instructions that when executed by a processor of a nucleic acid amplification system cause the nucleic acid amplification system to perform the method according to any one of claims 1 to 16.
